# EUROPEAN PATENT SPECIFICATION

(11) **EP 4 493 117 B1**
(45) Date of publication and mention of the grant of the patent: **08.07.2026**
(21) Application number: 23719223.2
(22) Date of filing: 15.03.2023
(51) Int. Cl.: A61F 2/00, A61F 2/26, F04B 43/04

(54) **FLUID CONTROL SYSTEM FOR AN IMPLANTABLE INFLATABLE DEVICE**
FLÜSSIGKEITSKONTROLLSYSTEM FÜR EINE IMPLANTIERBARE AUFBLASBARE VORRICHTUNG
SYSTÈME DE COMMANDE DE FLUIDE POUR UN DISPOSITIF GONFLABLE IMPLANTABLE

(30) Priority: 16.03.2022 US 202263269437 P; 13.03.2023 US 202318182612
(43) Date of publication of application: 22.01.2025
(73) Proprietor: Boston Scientific Scimed, Inc., Maple Grove, MN 55311-1566 (US)
(72) Inventor: MARCOS LARANGEIRA, Eduardo, Clonmel Tipperary, E91 W1R9 (IE); SMITH, Noel, Windgap, R95 HX88 (IE); NOLAN, Daragh, Via Youghal, P36YN35 (IE); SINNOTT, Thomas, Enniscorthy, Y21 (IE)
(74) Representative: Pfenning, Meinig & Partner mbB
(86) International application number: PCT/US2023/064395
(87) International publication number: WO 2023/178149

(56) References cited:
- WO-A1-2020/254435
- WO-A1-2020/254435
- US-A- 5 759 015
- US-A- 5 759 015
- US-A1- 2007 213 580
- US-A1- 2007 213 580
- US-A1- 2020 222 188
- US-A1- 2020 222 188
- US-B1- 6 247 908
- US-B1- 6 247 908

## Description

### TECHNICAL FIELD

This disclosure relates generally to bodily implants, and more specifically to bodily implants including a fluid control system having one or more pumps and/or valves including a piezoelectric actuator.

### BACKGROUND

Active implantable fluid operated inflatable devices often include one or more pumps that regulate a flow of fluid between different portions of the implantable device. One or more valves can be positioned within fluid passageways of the device to direct and control the flow of fluid to achieve inflation, deflation, pressurization, depressurization, activation, deactivation, and the like of different fluid filled implant components of the device. In some implantable fluid operated devices, an implantable pumping device may be manually operated by the user to provide for the transfer of fluid between a reservoir and the fluid filled implant components of the device. Manipulation of the manually operated implantable pumping device may be challenging for some patients. Further, such manual operation of the pumping device may make it may be difficult to achieve consistent inflation, deflation, pressurization, depressurization, activation, deactivation, and the like of the fluid filled implant components. Inconsistent inflation, deflation, pressurization, depressurization, activation and/or deactivation of the fluid filled implant device(s) may adversely affect patient comfort, efficacy of the device, and the overall patient experience. Accurate actuation and control of a fluid control system controlling the flow of fluid between components of the inflatable device will improve performance and efficacy of the device, and will improve patient comfort and safety.

Document US-A-2020/222188 discloses an implantable fluid operated inflatable device, comprising: a fluid reservoir; an inflatable member; and a fluid control system configured to control fluid flow between the fluid reservoir and the inflatable member, the fluid control system including: a housing; fluidic architecture defining one or more fluid passageways within in the housing; and at least one pump and at least one valve positioned in the one or more fluid passageways.

### SUMMARY

The present invention relates to an implantable fluid operated inflatable device includes a fluid reservoir, an inflatable member, and a fluid control system configured to control fluid flow between the fluid reservoir and the inflatable member. The fluid control system includes a housing, fluidic architecture defining one or more fluid passageways within in the housing, and at least one pump and at least one valve positioned in the one or more fluid passageways, the at least one pump and the at least one valve including a piezoelectric actuator that is operable in response to a voltage applied thereto by an electronic control system of the fluid control system. The piezoelectric actuator includes a diaphragm, and a piezoelectric element mounted on the diaphragm and configured to deform in response to a voltage applied thereto by the electronic control system. The diaphragm is configured to deform in response to deformation of the piezoelectric element mounted thereon. The piezoelectric actuator actuates the at least one pump and the at least one valve to control a flow of fluid in the one or more fluid passageways based on one of an amount of the deformation of the diaphragm or a direction of the deformation of the diaphragm.

In some implementations, the piezoelectric element includes a plate portion mounted on the diaphragm, and an open portion in the plate portion such that the plate portion surrounds the open portion. A position of the open portion of the piezoelectric element may correspond to a high bending stress region of the piezoelectric actuator during deformation of the piezoelectric actuator. The open portion of the piezoelectric element may be defined by a substantially circular opening at a central portion of the piezoelectric element, and the plate portion forms an annular ring surrounding the open portion.

In some implementations, the plate portion includes a plurality of separate segments arranged surrounding the opening. Each of the plurality of separate segments may be separately actuatable in response to a voltage applied thereto by the electronic control system of the fluid control system. A size and a shape of each of the plurality of separate segments may be substantially the same. The plurality of separate segments may be arranged substantially symmetrically about a central plane of the piezoelectric element.

In some implementations, the piezoelectric actuator includes a protrusion extending along a peripheral portion of the diaphragm. A contour of the protrusion may correspond to an outer peripheral contour of the plate portion of the piezoelectric element such that the piezoelectric element is received within an area of the diaphragm enclosed by the protrusion.

In some implementations, the piezoelectric actuator includes a piezoelectric element configured to deform in response to a voltage applied thereto by an electronic control system of the fluid control system, a first plate coupled to a first side of the piezoelectric element, the first plate having a convex contour relative to the first side of the piezoelectric element, a second plate coupled to a second side of the piezoelectric element, the second side being opposite the first side of the piezoelectric element, the second plate having a convex contour relative to the second side of the piezoelectric element, and a diaphragm coupled to the second plate. The diaphragm may be configured to deform in response to deformation of the piezoelectric element. A distance between a central portion of the first plate and the diaphragm may be greater than a distance between peripheral portions of the first plate and the diaphragm, and a distance between a central portion of the second plate and the diaphragm may be greater than a distance between peripheral portions of the second plate and the diaphragm. Peripheral edge portions of the first plate may be coupled to peripheral edge portions of the first side of the piezoelectric element, peripheral edge portions of the second plate may be coupled to peripheral edge portions of the second side of the piezoelectric element, and the diaphragm may be coupled to a central portion of a central portion of the second plate, with the second plate positioned between the diaphragm and the piezoelectric element.

In some implementations, the piezoelectric element is configured to expand in a planar direction in response to a first voltage applied thereto, and to contract in the planar direction in response to a second voltage applied thereto, and the first plate and the second plate are configured to deform in response to expansion or contraction of the piezoelectric element. In some implementations, the first plate is configured to deform in a first direction and the second plate is configured to deform in a second direction opposite the first direction in response to expansion of the piezoelectric element to decrease a distance between the first plate and the second plate; the first plate is configured to deform in the second direction and the second plate is configured to deform in the first direction in response to contraction of the piezoelectric element to increase a distance between the first plate and the second plate; the diaphragm is configured to deform in the first direction in response to expansion of the piezoelectric element; and the diaphragm is configured to deform in the second direction in response to contraction of the piezoelectric element.

In some implementations, the piezoelectric element includes a stack of piezoelectric layers, including a plurality of piezoelectric layers arranged sequentially along a mounting surface of the diaphragm, from a first end portion to a second end portion of the stack of piezoelectric layers, a first support bracket coupling a first piezoelectric layer, at the first end portion of the stack of piezoelectric layers, to a mounting surface of the diaphragm, and a second support bracket coupling a second piezoelectric layer, at the second end portion of the stack of piezoelectric layers, to the mounting surface of the diaphragm. The stack of piezoelectric layers may be configured to expand in response to a first voltage applied thereto, the first end portion of the stack of piezoelectric layers may be configured to pivot in a first pivotal direction at the first support bracket in response to expansion of the stack of piezoelectric layers, and the second end portion of the stack of piezoelectric layers may be configured to pivot in a second pivotal direction at the second support bracket in response to expansion of the stack of piezoelectric layers. The stack of piezoelectric layers may be configured to contract in response to a second voltage applied thereto, the first end portion of the stack of piezoelectric layers may be configured to pivot in the second pivotal direction at the first support bracket in response to contraction of the stack of piezoelectric layers, and the second end portion of the stack of piezoelectric layers may be configured to pivot in the first pivotal direction at the second support bracket in response to contraction of the stack of piezoelectric layers. The diaphragm may be configured to deform in a first direction in response to expansion of the stack of piezoelectric layers. The diaphragm may be configured to deform in a second direction in response to contraction of the stack of piezoelectric layers. A space may be formed between a bottom portion of the piezoelectric element and the mounting surface of the diaphragm, and a distance between the bottom portion of the piezoelectric element and the mounting surface of the diaphragm may increase in response to expansion of the stack of piezoelectric layers, and may decrease in response to contraction of the stack of piezoelectric layers.

In another general aspect, an implantable fluid operated inflatable device includes a fluid reservoir, an inflatable member, and a fluid control system configured to control fluid flow between the fluid reservoir and the inflatable member The fluid control system may include a housing, fluidic architecture defining one or more fluid passageways within in the housing, and at least one pump and at least one valve positioned in the one or more fluid passageways, the at least one pump and the at least one valve including a piezoelectric actuator that is operable in response to a voltage applied thereto by an electronic control system of the fluid control system. The piezoelectric actuator may include a diaphragm mounted in a fluid passageway of the one or more fluid passageways defined within the housing to control a flow of fluid through the fluid passageway, and a piezoelectric element mounted on the diaphragm and configured to deform in response to a voltage applied thereto by the electronic control system. The diaphragm may be configured to deform in response to deformation of the piezoelectric element mounted thereon. The piezoelectric element may include a plate portion mounted on the diaphragm, and an open portion in the plate portion such that the plate portion surrounds the open portion.

In some implementations, the open portion of the piezoelectric element is defined by a substantially circular opening at a central portion of the piezoelectric element. The plate portion forms an annular ring surrounding the open portion. The plate portion may include a plurality of separate segments arranged surrounding the opening. A position of the open portion of the piezoelectric element may correspond to a high bending stress region of the piezoelectric actuator during deformation of the piezoelectric actuator. In some implementations, each of the plurality of separate segments is separately actuatable in response to a voltage applied thereto by the electronic control system of the fluid control system. In some implementations, at least one of the plurality of separate segments is actuatable in response to a voltage applied thereto by the electronic control system of the fluid control system to control the flow of fluid through the fluid passageway, and at least one of the plurality of separate segments is configured to sense a pressure of fluid in the fluid passageway. In some implementations, a size and a shape of each of the plurality of separate segments is substantially the same, and the plurality of separate segments is arranged substantially symmetrically about a central plane of the piezoelectric element.

In some implementations, the diaphragm includes a protrusion extending along a peripheral portion of the diaphragm. A contour of the protrusion may correspond to an outer peripheral contour of the plate portion of the piezoelectric element such that the piezoelectric element is received within an area of the diaphragm enclosed by the protrusion.

In another general aspect, an implantable fluid operated inflatable device may include a fluid reservoir, an inflatable member, and a fluid control system coupled between the fluid reservoir and the inflatable member and configured to control fluid flow between the fluid reservoir and the inflatable member. The fluid control system may include a housing, a fluid control system including fluidic architecture defining one or more fluid passageways within in the housing, and at least one pump and at least one valve positioned in the one or more fluid passageways, the at least one pump and the at least one valve including a piezoelectric actuator that is operable in response to a voltage applied thereto by an electronic control system of the fluid control system. In some implementations, the piezoelectric actuator includes a piezoelectric element configured to deform in response to a voltage applied thereto by an electronic control system of the fluid control system, a first plate coupled to a first side of the piezoelectric element, the first plate having a convex contour relative to the first side of the piezoelectric element, a second plate coupled to a second side of the piezoelectric element, the second side being opposite the first side of the piezoelectric element, the second plate having a convex contour relative to the second side of the piezoelectric element, and a diaphragm coupled to the second plate, wherein the diaphragm is configured to deform in response to deformation of the piezoelectric element.

In some implementations, a distance between a central portion of the first plate and the piezoelectric element is greater than a distance between peripheral portions of the first plate and the piezoelectric element, and a distance between a central portion of the second plate and the piezoelectric element is greater than a distance between peripheral portions of the second plate and the piezoelectric element. In some implementations, peripheral edge portions of the first plate are coupled to peripheral edge portions of the first side of the piezoelectric element, peripheral edge portions of the second plate are coupled to peripheral edge portions of the second side of the piezoelectric element, and the diaphragm is coupled to a central portion of a central portion of the second plate, with the second plate positioned between the diaphragm and the piezoelectric element. The piezoelectric element may be configured to expand in a planar direction in response to a first voltage applied thereto, and to contract in the planar direction in response to a second voltage applied thereto. The first plate and the second plate may be configured to deform in response to expansion or contraction of the piezoelectric element.

In some implementations, the first plate is be configured to deform in a first direction and the second plate is configured to deform in a second direction opposite the first direction in response to expansion of the piezoelectric element to decrease a distance between the first plate and the second plate. In some implementations, the first plate is configured to deform in the second direction and the second plate is configured to deform in the first direction in response to contraction of the piezoelectric element to increase a distance between the first plate and the second plate. The diaphragm may be configured to deform in the first direction in response to expansion of the piezoelectric element, and the diaphragm may be configured to deform in the second direction in response to contraction of the piezoelectric element.

In another general aspect, an implantable fluid operated inflatable device may include a fluid reservoir, an inflatable member, and a fluid control system configured to control fluid flow between the fluid reservoir and the inflatable member. The fluid control system may include a housing, fluidic architecture defining one or more fluid passageways within in the housing, and at least one pump and at least one valve positioned in the one or more fluid passageways, the at least one pump and the at least one valve including a piezoelectric actuator that is operable in response to a voltage applied thereto by an electronic control system of the fluid control system. The piezoelectric actuator may include a diaphragm mounted in a fluid passageway of the one or more fluid passageways defined within the housing to control a flow of fluid through the fluid passageway, and a piezoelectric element mounted on the diaphragm and configured to deform in response to a voltage applied thereto by an electronic control system of the fluid control system. The diaphragm may be configured to deform in response to deformation of the piezoelectric element mounted thereon. The piezoelectric element may include a stack of piezoelectric layers, including a plurality of piezoelectric layers arranged sequentially from a first end portion to a second end portion of the stack of piezoelectric layers, a first support bracket coupling a first piezoelectric layer, at the first end portion of the stack of piezoelectric layers, to a mounting surface of the diaphragm, and a second support bracket coupling a second piezoelectric layer, at the second end portion of the stack of piezoelectric layers, to the mounting surface of the diaphragm.

In some implementations, the plurality of piezoelectric layers may be arranged sequentially along the mounting surface of the diaphragm, from the first end portion to the second end portion of the stack of piezoelectric layers. The stack of piezoelectric layers may be configured to expand in response to a first voltage applied thereto. The first end portion of the stack of piezoelectric layers may be configured to pivot in a first pivotal direction at the first support bracket in response to expansion of the stack of piezoelectric layers, and the second end portion of the stack of piezoelectric layers may be configured to pivot in a second pivotal direction at the second support bracket in response to expansion of the stack of piezoelectric layers. In some implementations, the stack of piezoelectric layers is configured to contract in response to a second voltage applied thereto, the first end portion of the stack of piezoelectric layers is configured to pivot in the second pivotal direction at the first support bracket in response to contraction of the stack of piezoelectric layers, and the second end portion of the stack of piezoelectric layers is configured to pivot in the first pivotal direction at the second support bracket in response to contraction of the stack of piezoelectric layers. In some implementations, the diaphragm is configured to deform in a first direction in response to expansion of the stack of piezoelectric layers, and the diaphragm is configured to deform in a second direction in response to contraction of the stack of piezoelectric layers. In some implementations, a space is formed between a bottom portion of the piezoelectric element and the mounting surface of the diaphragm, and a distance between the bottom portion of the piezoelectric element and the mounting surface of the diaphragm increases in response to expansion of the stack of piezoelectric layers, and decreases in response to contraction of the stack of piezoelectric layers.

### BRIEF DESCRIPTION OF THE DRAWINGS

FIG. 1 is a block diagram of an implantable fluid operated inflatable device according to an aspect.
FIG. 2A illustrates a first system including a first example implantable fluid operated inflatable device according to an aspect.
FIG. 2B illustrates a second system including a second example implantable fluid operated inflatable device according to an aspect.
FIG. 3 is a schematic diagram of a fluidic architecture of an implantable fluid operated inflatable device according to an aspect.
FIG. 4A is a schematic view of an example active valve, in an open state.
FIG. 4B is a schematic view of the example active valve shown in FIG. 4A, in a closed state.
FIG. 5A is a top perspective view of an example piezoelectric actuator, according to an aspect.
FIG. 5B is a cross-sectional view of the example piezoelectric actuator shown in FIG. 5A, taken along line A-A of FIG. 5A.
FIG. 5C illustrates the example piezoelectric actuator shown in FIGs. 5A and 5B in a first actuated state.
FIG. 5D illustrates the example piezoelectric actuator shown in FIGs. 5A and 5B in a second actuated state.
FIG. 6 is a top perspective view of an example piezoelectric actuator, according to an aspect.
FIG. 7A is a side view of an example piezoelectric actuator, according to an aspect.
FIG. 7B is cross-sectional view of the example piezoelectric actuator shown in FIG. 7A, taken along line B-B of FIG. 5A.
FIG. 7C is a cross-sectional view of the example piezoelectric actuator shown in FIG. 7A, coupled to a diaphragm.
FIG. 7D illustrates the example piezoelectric actuator shown in FIGs. 7A-7C in a first actuated state.
FIG. 7E illustrates the example piezoelectric actuator shown in FIGs. 7A-7C in a second actuated state.
FIG. 8A is a top perspective view of an example piezoelectric actuator, according to an aspect.
FIG. 8B illustrates the example piezoelectric actuator shown in FIG. 8A in a first actuated state.
FIG. 8C illustrates the example piezoelectric actuator shown in FIG. 8A in a second actuated state.
FIG. 9A is a top perspective view of an example piezoelectric actuator, according to an aspect.
FIG. 9B is a cross-sectional view of the example piezoelectric actuator shown in FIG. 9A, taken along line H-H of FIG. 9A.

### DETAILED DESCRIPTION

Detailed implementations are disclosed herein. However, it is understood that the disclosed implementations are merely examples, which may be embodied in various forms. Therefore, specific structural and functional details disclosed herein are not to be interpreted as limiting, but merely as a basis for the claims and as a representative basis for teaching one skilled in the art to variously employ the implementations in virtually any appropriately detailed structure. Further, the terms and phrases used herein are not intended to be limiting, but to provide an understandable description of the present disclosure.

The terms "a" or "an," as used herein, are defined as one or more than one. The term "another," as used herein, is defined as at least a second or more. The terms "including" and/or "having", as used herein, are defined as comprising (i.e., open transition). The term "coupled" or "moveably coupled," as used herein, is defined as connected, although not necessarily directly and mechanically.

In general, the implementations are directed to bodily implants. The term patient or user may hereinafter be used for a person who benefits from the medical device or the methods disclosed in the present disclosure. For example, the patient can be a person whose body is implanted with the medical device or the method disclosed for operating the medical device by the present disclosure.

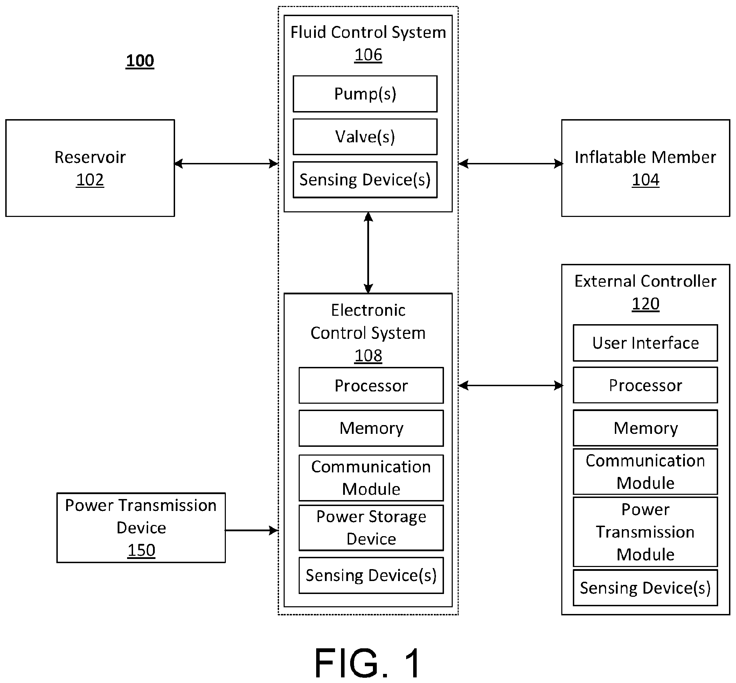

FIG. 1 is a block diagram of an example implantable fluid operated inflatable device 100. The example device 100 shown in FIG. 1 includes a fluid reservoir 102, an inflatable member 104, and a fluid control system 106. The fluid control system 106 can include fluidics components such as one or more pumps, one or more valves and the like configured to transfer fluid between the fluid reservoir 102 and the inflatable member 104. The fluid control system 106 can include one or more sensing devices that sense conditions such as, for example, fluid pressure, fluid flow rate and the like within the fluidics architecture of the inflatable device 100. In some implementations, the inflatable device 100 includes an electronic control system 108. The electronic control system 108 may provide for the monitoring and/or control of the operation of various fluidics components of the fluid control system 106 and/or communication with one or more sensing device(s) within the implantable fluid operated inflatable device 100 and/or communication with one or more external device(s). In some examples, the electronic control system 108 includes components such as a processor, a memory, a communication module, a power storage device, or battery, sensing devices such as, for example an accelerometer, and other such components configured to provide for the operation and control of the implantable fluid operated inflatable device 100. In some examples, the communication module of the electronic control system 108 may provide for communication with one or more external devices such as, for example, an external controller 120.

In some examples, the external controller 120 includes components such as, for example, a user interface, a processor, a memory, a communication module, a power transmission module, and other such components providing for operation and control of the external controller 120 and communication with the electronic control system 108 of the inflatable device 100. For example, the memory may store instructions, applications and the like that are executable by the processor of the external controller 120. The external controller 120 may be configured to receive user inputs via, for example, the user interface, and to transmit the user inputs, for example, via the communication module, to the electronic control system 108 for processing, operation and control of the inflatable device 100. Similarly, the electronic control system 108 may, via the respective communication modules, transmit operational information to the external controller 120. This may allow operational status of the inflatable device 100 to be provided, for example, through the user interface of the external controller 120, to the user, may allow diagnostics information to be provided to a physician, and the like.

In some examples, the power transmission module of the external controller 120 provides for charging of the components of the internal electronic control system 108. In some examples, transmission of power for the charging of the internal electronic control system 108 can be, alternatively or additionally, provided by an external power transmission device 150 that is separate from the external controller 120. In some implementations the external controller 120 can include sensing devices such as a pressure sensor, an accelerometer and other such sensing devices. An external pressure sensor in the external controller 120 may provide, for example, a local atmospheric or working pressure to the internal electronic control system 108, to allow the inflatable device 100 to compensate for variations in pressure. An accelerometer in the external controller 120 may provide detected patient movement to the internal electronic control system 108 for control of the inflatable device 100.

The fluid reservoir 102, the inflatable member 104, the fluid control system 106 and the electronic control system 108 may be internally implanted into the body of the patient. In some implementations, the electronic control system 108 is coupled to or incorporated into a housing of the fluid control system 106. In some implementations, at least a portion of the electronic control system 108 is physically separate from the fluid control system 106. In some implementations, some modules of the electronic control system 108 are coupled to or incorporated into the fluid control system 106, and some modules of the electronic control system 108 are separate from the fluid control system 106. For example, in some implementations, some modules of the electronic control system 108 are included in an external device (such as the external controller 120) that is in communication other modules of the electronic control system 108 included within the implantable device 100. In some implementations, at least some aspects of the operation of the implantable fluid operated inflatable device 100 may be manually controlled.

In some examples, electronic monitoring and control of the fluid operated inflatable device 100 may provide for improved patient control of the device, improved patient comfort, and improved patient safety. In some examples, electronic monitoring and control of the fluid operated device 100 may afford the opportunity for tailoring of the operation of the inflatable device 100 by the physician without further surgical intervention. Fluidic architecture defining the flow and control of fluid through the fluid operated inflatable device 100, including the configuration and placement of fluidics components such as pumps, valves, sensing devices and the like, may allow the inflatable device 100 to precisely monitor and control operation of the inflatable device, effectively respond to user inputs, and quickly and effectively adapt to changing conditions both within the inflatable device 100 (changes in pressure, flow rate and the like) and external to the inflatable device 100 (pressure surges due to physical activity, impacts and the like, sustained pressure changes due to changes in atmospheric conditions, and other such changes in external conditions).

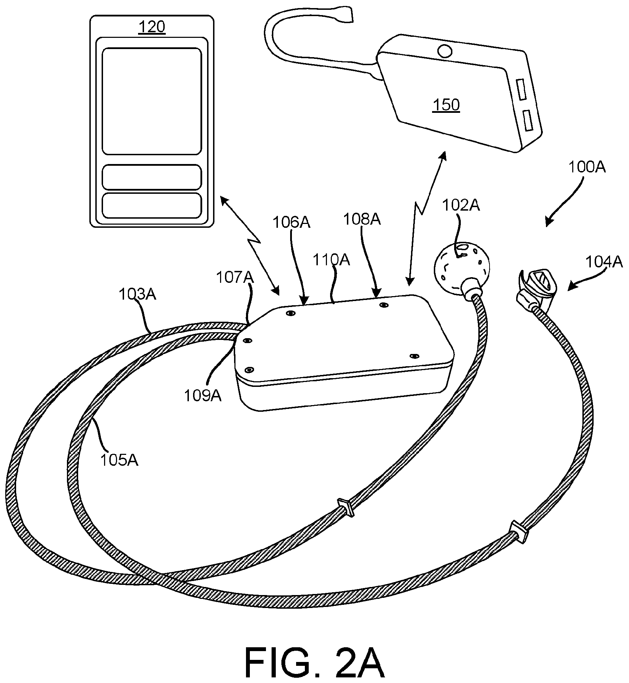

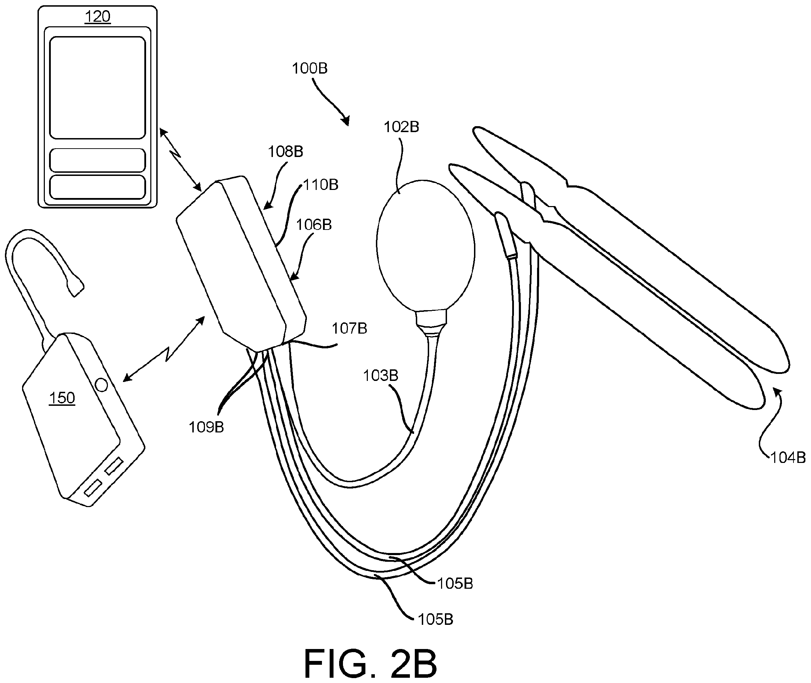

The example implantable fluid operated inflatable device 100 may be representative of a number of different types of implantable fluid operated devices. For example, the device 100 shown in FIG. 1 may be representative of an artificial urinary sphincter 100A as shown in FIG. 2A, an inflatable penile prosthesis 100B as shown in FIG. 2B, and other such implantable inflatable devices that rely on the control of fluid flow to components of the device to achieve inflation, pressurization, deflation, depressurization, deactivation, and the like.

A first example system including a first example implantable fluid operated inflatable device in the form of an example artificial urinary sphincter 100A is shown in FIG. 2A. The artificial urinary sphincter 100A includes a fluid control system 106A including fluidics components such as pumps, valves, sensing devices and the like positioned in fluid passageways, and an electronic control system 108A configured to provide for the transfer of fluid between a reservoir 102A and an inflatable cuff 104A via the fluidics components. Fluidics components of the fluid control system 106A, and electronic components of the electronic control system 108A may be received in a housing 110A. A first conduit 103A connects a first fluid port 107A of the fluid control system 106A/electronic control system 108A received in the housing 110A with the reservoir 102A. A second conduit 105A connects a second fluid port 109A of the fluid control system 106A/electronic control system 108A received in the housing 110A with the inflatable cuff 104A. The electronic control system 108A of the artificial urinary sphincter 100A can communicate with the external controller 120, via the respective communication modules. For example, an application stored in the memory and executed by the processor of the external controller 120 may allow the user and/or a physician to operate, view, monitor and alter operation of the artificial urinary sphincter 100A. In some examples, components of the electronic control system 108A and/or the fluid control system 106A may be charged and/or recharged by a power transmission module of the external controller 120, and/or by a power transmission device 150, that is separate from the external controller 120.

A second example system including a second example implantable fluid operated inflatable device in the form of an example penile prosthesis 100B is shown in FIG. 2B. The penile prosthesis 100B includes a fluid control system 106B including fluidics components such as pumps, valves, sensing devices and the like positioned in fluid passageways, and an electronic control system 108B configured to provide for the transfer of fluid between a fluid reservoir 102B and inflatable cylinders 104B via the fluidics components. Fluidics components of the fluid control system 106B, and electronic components of the electronic control system 108B may be received in a housing 110B. A first conduit 103B connects a first fluid port 107B of the fluid control system 106B/electronic control system 108B received in the housing 110B with the reservoir 102B. One or more second conduits 105B connect one or more second fluid ports 109B of the fluid control system 106A/electronic control system 108A received in the housing with the inflatable cylinders 104B. The electronic control system 108A of the penile prosthesis 100B can communicate with the external controller 120, via the respective communication modules. For example, an application stored in the memory and executed by the processor of the external controller 120 may allow the user and/or a physician to operate, view, monitor and alter operation of the penile prosthesis. In some examples, components of the electronic control system 108A and/or the fluid control system 106A may be charged and/or recharged by a power transmission module of the external controller 120, and/or by a power transmission device 150, that is separate from the external controller 150

The principles to be described herein may be applied to the example implantable fluid operated inflatable devices shown in FIGs. 2A and 2B, and other types of implantable fluid operated inflatable devices that rely on a pump assembly including various fluidics components to provide for the transfer of fluid between the different fluid filled implantable components to achieve inflation, deflation, pressurization, depressurization, deactivation, occlusion, and the like for effective operation. The example inflatable devices 100A, 100B shown in FIGS. 2A and 2B include electronic control systems 108A, 108B to provide for control of the operation of the respective inflatable members 104A, 104B, and the monitoring and control of pressure and/or fluid flow through the respective inflatable devices 100A, 100B. Some of the principles to be described herein may also be applied to implantable fluid operated inflatable devices that are manually controlled.

As noted above, the fluid control system 106 (106A, 106B) can include a pump assembly including, for example, one or more pumps and one or more valves positioned within a fluid circuit of the pump assembly to control the transfer fluid between the fluid reservoir 102 (102A, 102B) and the inflatable member 104 (104A, 104B). In some examples, the pump(s) and/or the valve(s) are electronically controlled. In some examples, the pump(s) and/or the valve(s) are manually controlled. In an example in which the pump assembly is electronically powered and/or controlled, the pump assembly may include a hermetic manifold that can contain and segment the flow of fluid from electronic components of the pump assembly, to prevent leakage and/or gas exchange. In some examples, the pump(s) and/or valve(s) may include piezoelectric elements. In some examples, the pump assembly includes one or more pressure sensing devices in the fluid circuit to provide for relatively precise monitoring and control of fluid flow and/or fluid pressure within the fluid circuit and/or the inflatable member. A fluid circuit configured in this manner may facilitate the proper inflation, deflation, pressurization, depressurization, and deactivation of the components of the implantable fluid operated device to provide for patient safety and device efficacy.

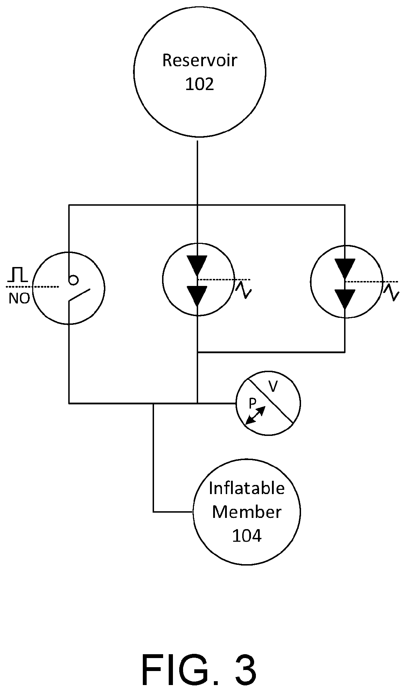

FIG. 3 is a schematic diagram of an example fluidic architecture for an implantable fluid operated inflatable device, according to an aspect. The fluidic architecture of an implantable fluid operated inflatable device can include other arrangements of fluidic channels, valve(s), pressure sensor(s) and other components than shown in FIG. 3. The example fluidic architecture shown in FIG. 3 includes channels guiding the flow of fluid between the reservoir 102 and the inflatable member 104. In some examples, one or more of the valves included in the fluidic architecture are normally open valves. Normally open valves default to an open state, and close (and remain closed) in response to the application of power. In some examples, one or more of the valves included in the fluidic architecture may be normally closed valves, which default to the closed state, and open (and remain open) in response to the application of power.

In the example arrangement shown in FIG. 3, an active valve, or a normally open (NO) valve and first and second pumps are arranged in parallel in respective fluidic channels between the reservoir 102 and the inflatable member 104. A pressure sensing device positioned at the inflatable member 104 monitors a pressure at the inflatable member 104. During an inflation operation, the pumps operate to transfer fluid from the reservoir 102 to the inflatable member 104 to cause inflation of the inflatable member 104. Power is applied to the normally open valve during the inflation operation to close the normally open valve, to prevent backflow, or the flow of fluid back towards the reservoir 102 and maintain the desired pressure at the inflatable member 104. During a deflation operation, power is no longer applied to the normally open valve, and the normally open valve defaults to the normal, open state. The open state of the normally open valve allows fluid to flow from the inflatable member 104 towards the reservoir 102 to achieve deflation of the inflatable member 104.

In some examples, the use of normally open valves may provide failsafe measures in the event of, for example, power failure or other system failure which would result in the loss of control of the pumps and/or valves. For example, a loss of power (or other system failure) in state in which the inflatable member 104 is inflated could cause patient discomfort and/or compromise patient safety. The use of normally open valves in the fluidics architecture allows for the flow of fluid from the inflatable member 104 towards the reservoir 102 in the event of a power loss, thus relieving pressure from the inflatable member 104, and for the fluid in the system to reach equilibrium.

Normally closed may not provide these types of failsafe measures, but may reduce power consumption of the fluid operated inflatable device 100. That is, normally closed valves default to the closed state and do not rely on the application of power to remain in the closed state. As many of the valves in the fluidic architecture remain in the closed state for considerably more time than in the open state (for example, to maintain a current state of the fluid operated inflatable device 100), the use of one or more normally closed valves the fluidic architecture may reduce power consumption (when compared to the use of normally open valves). This may increase longevity of the fluid operated inflatable device 100, reduce physician intervention required for continued operation (to, for example, replace power cells), and/or reduce re-charging requirements and/or increase intervals between re-charging.

As described above, example fluidic architecture for an implantable fluid operated inflatable device, according to an aspect may include one or more pumps and one or more valves that operate to transfer fluid between the reservoir 102 and the inflatable member 104. In a pressurization or inflation operation, the one or more pumps and one or more valves operate to transfer fluid from the reservoir 102 to the inflatable member, in a predetermined amount of time, to reach a set pressure at the inflatable member 104. During the pressurization or inflation operation, the active valve (a normally open valve in the example arrangement shown in FIG. 3) remains closed, preventing recirculation of fluid back toward the inlet(s) of the pump(s) and the reservoir 102. For depressurization or deflation from the pressurized or inflated state, the active/normally open valve is opened (for example, in response to a user input), opening the fluidic channel and allowing fluid to flow out of the inflatable member 104 and back towards the reservoir 102.

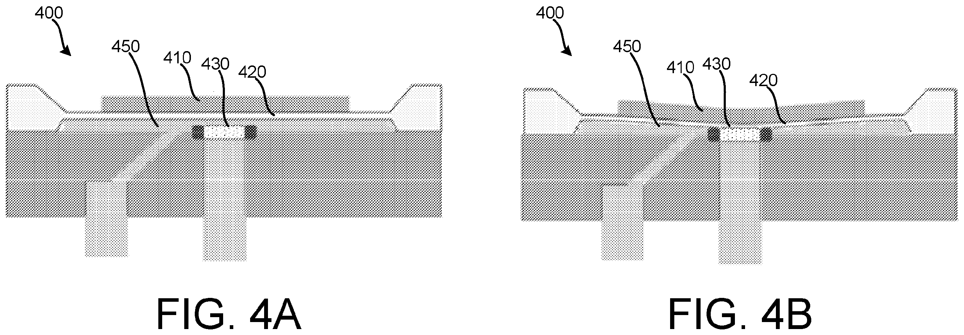

In some examples, a normally open active valve employs a piezoelectric element that acts on a diaphragm. FIGs. 4A and 4A schematically illustrate the operation of a normally open active valve 400 employing a piezoelectric element, or a piezoelectric actuator. In particular, FIG. 4A illustrates the open state of the normally open active valve 400, and FIG. 4B illustrates the closed state of the normally open active valve 400. The principles to be described herein may be similarly applied to the operation and control of a normally closed valve including a piezoelectric actuator, the operation and control of a pump including a piezoelectric actuator, the operation of a combination pump and valve including a piezoelectric actuator, and the like.

The example normally open active valve 400 shown in FIGs. 4A and 4B includes a piezoelectric element 410 in the form of a disc made of a piezoelectric material (for example, a piezo-ceramic disc) mounted on a diaphragm 420. In the arrangement shown in FIG. 4A, the normally open active valve 400 is in a default state, or at rest state, or open state, in which fluid can flow through a chamber 450, i.e., from an inlet to an outlet of the chamber 450. In FIG. 4B, the normally open active valve 400 has transitioned to a closed state in response to actuation (for example, application of power to the piezoelectric disc 410). In the arrangement shown in FIG. 4B, the application of power to the piezoelectric disc 410 has caused deformation or deflection of the piezoelectric disc 410, and corresponding deformation or deflection of the diaphragm 420. In the deformed state, the deformed piezoelectric disc 410 and diaphragm 420 press against a sealing element 430 (such as, for example, an O-ring), to close or seal the fluid flow path between the inlet and the outlet of the chamber 450. The principles to be described herein may be similarly applied to valves and to pumps of the fluidic architecture of an implantable fluid operated inflatable device according to an aspect.

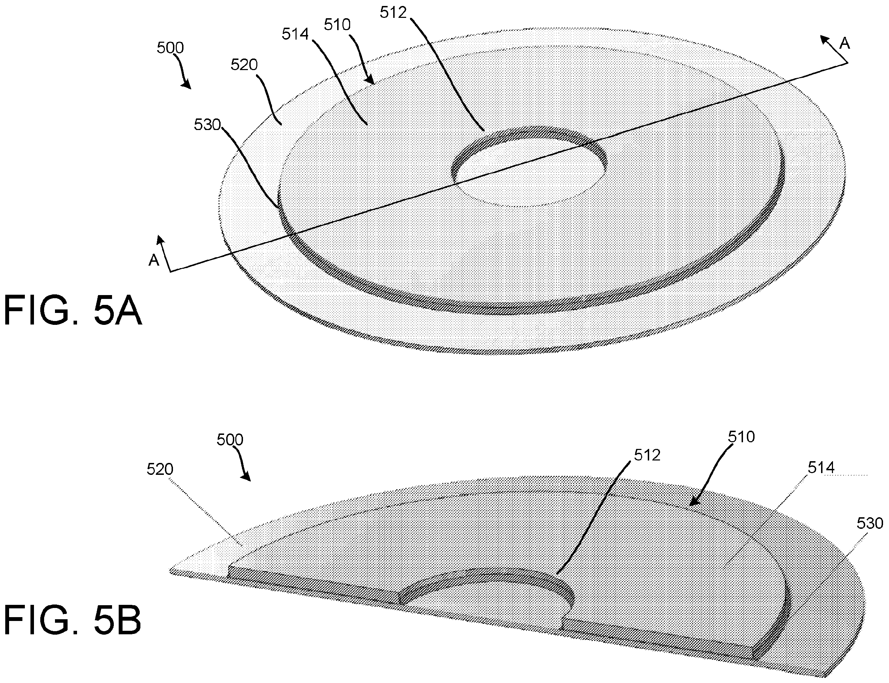

FIG. 5A is a top perspective view, and FIG. 5B is a cross sectional view, of an example piezoelectric actuator 500, according to an aspect. The example piezoelectric actuator 500 shown in FIGs. 5A and 5B may be used in a valve and/or a pump of the fluidic architecture of an implantable fluid operated inflatable device according to an aspect.

As shown in FIGs. 5A and 5B, the piezoelectric actuator 500 includes a piezoelectric element 510, in the form of a piezoelectric ring 510 mounted on a diaphragm 520. In some examples, an adhesive layer 530, or an epoxy layer 530, is disposed between the piezoelectric ring 510 and the diaphragm 520 to couple the piezoelectric ring 510 on the diaphragm 520. The piezoelectric ring 510 includes a plate portion 514 having an open portion 512 formed at a central portion thereof. In the example shown in FIGs. 5A and 5B, the plate portion 514 has an annular shape or configuration, surrounding the substantially circular open portion 512 of the piezoelectric ring 510. In the example shown in FIGs. 5A and 5B, the piezoelectric actuator 500, the piezoelectric ring 510 (including the open portion 512 and the plate portion 514) and the diaphragm 520 are substantially circular, and are all substantially concentrically arranged, simply for purposes of discussion and illustration. The principles to be described herein may be applied to piezoelectric rings, open portions, plate portions, and diaphragms having other shapes and/or contours and/or combinations of shapes/contours.

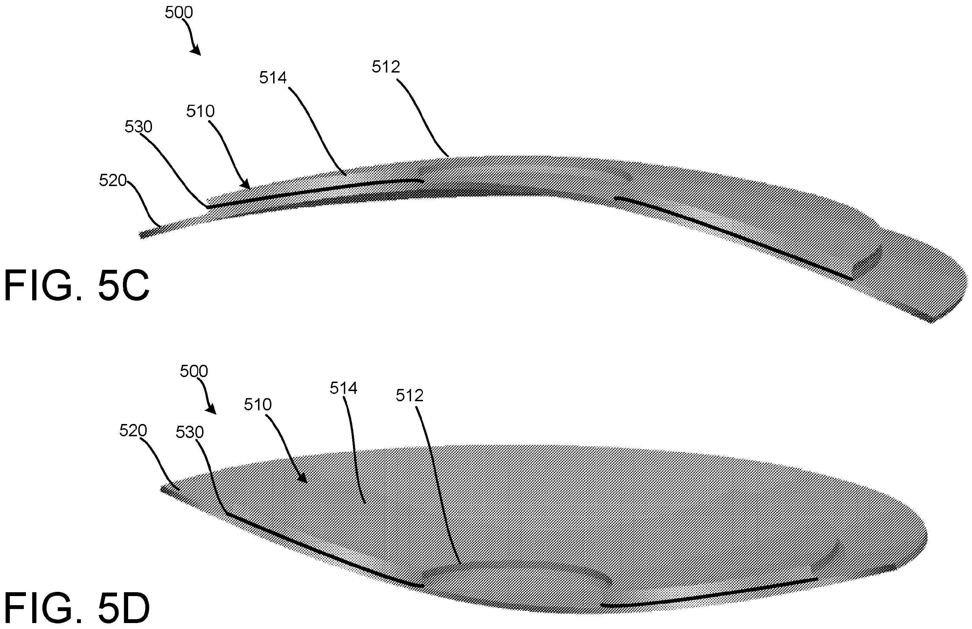

As described above, the piezoelectric ring 510 will bend or deform in response to the application of power, or voltage, causing the diaphragm 520 to also bend or deform due to the radial strain applied to the diaphragm 520 by the bending or deformation or deflection of the piezoelectric ring 510 attached thereto. FIG. 5C illustrates an upward displacement (in the example orientation shown in FIG. 5C) in response to the application of voltage having a first polarity. FIG. 5D illustrates a downward displacement (in the example orientation shown in FIG. 5D) in response to the application of voltage having a second polarity (opposite the first polarity).

In a piezoelectric element defined by a piezoelectric disc, a central region of the piezoelectric disc would represent a relatively high stress region of the piezoelectric disc as the piezoelectric discs bends and/or deforms in response to the application of voltage. That is, during bending or deformation of the piezoelectric actuator, the central portion of the piezoelectric disc would experience the greatest level of bending or deformation, and thus experience the greatest level of bending stresses during deformation. In contrast, the piezoelectric ring 510 shown in FIGs. 5A-5D includes the open portion 512 defined at the central region of the piezoelectric ring 510, corresponding to this relatively high stress region. When configured in this manner, as the piezoelectric ring 510 bends, bending or deformation or deflection of the plate portion 514 causes corresponding bending or deformation or deflection in the diaphragm 520 as shown, while the relatively high stresses that would otherwise be experienced at the central region (of a piezoelectric disc) during bending/deformation/deflection can be avoided due to the open portion 512 at the central region of the piezoelectric ring 510. Avoidance of the high stresses that would otherwise be experienced at the central region of the piezoelectric element during bending/deformation/deflection may improve performance and/or durability and/or reliability of the pump or valve in which the piezoelectric actuator 500 is installed.

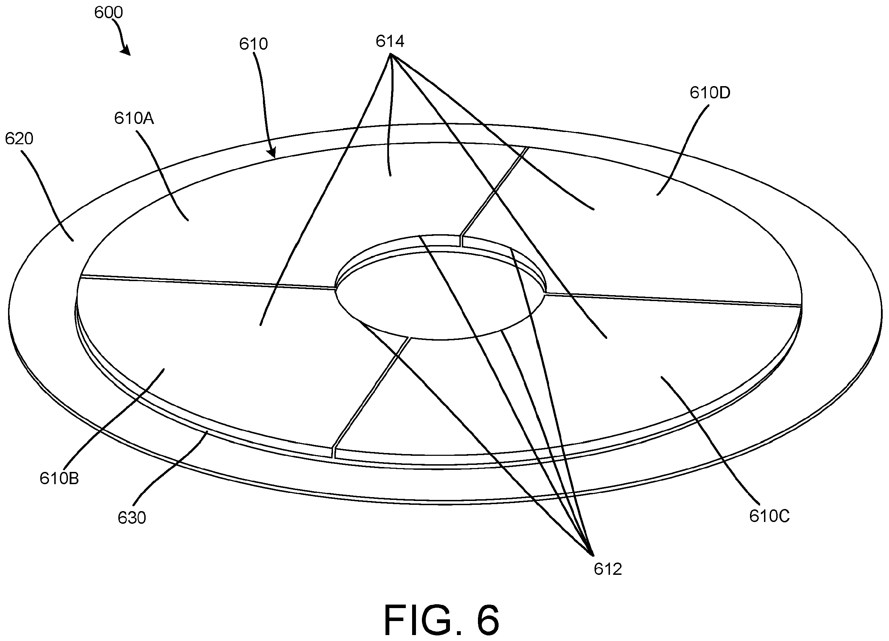

FIG. 6 is a top perspective view of an example piezoelectric actuator 600, according to an aspect. The example piezoelectric actuator 600 shown in FIG. 6 may be used in a valve and/or a pump of the fluidic architecture of an implantable fluid operated inflatable device according to an aspect.

As shown in FIG. 6, the piezoelectric actuator 600 includes a piezoelectric element 610, in the form of a piezoelectric ring 610 mounted on a diaphragm 620. In some examples, an adhesive layer 630, or an epoxy layer 630, is disposed between the piezoelectric ring 610 and the diaphragm 620 to couple the piezoelectric ring 610 on the diaphragm 620. The piezoelectric ring 610 includes an open portion 612 at a central portion thereof, with a plate portion 614 of the piezoelectric ring 610 surrounding the open portion 612. In the example shown in FIG. 6, the plate portion 614 has a substantially annular shape or configuration. In the example shown in FIG. 6, the piezoelectric actuator 600, the piezoelectric ring 610 (including the open portion 612 and the plate portion 614) and the diaphragm 620 are substantially circular, and substantially concentrically arranged, simply for purposes of discussion and illustration. The principles to be described herein may be applied to piezoelectric rings, open portions, plate portions, and diaphragms having other shapes and/or contours and/or combinations of shapes/contours.

As described above, the plate portion 614 of the piezoelectric ring 610 will bend or deform in response to the application of power, or voltage, causing the diaphragm 620 to also bend or deform. The relatively high stresses that would otherwise be experienced at the central region (of a piezoelectric disc) during bending or deformation or deflection can be avoided due to the open portion 612 at the central region of the piezoelectric ring 610, thus improving performance and/or durability and/or reliability of the pump or valve in which the piezoelectric actuator 600 is installed.

In the example arrangement shown in FIG. 6, the piezoelectric ring 610 is separated into segments 610A, 610B, 610C and 610D. The splitting of the piezoelectric ring 610 (i.e., the plate portion 614 of the piezoelectric ring 610 in this example) into separate segments 610A, 610B, 610C and 610D allows for independent movement of the separate segments 610A, 610B, 610C and 610D, and for independent or individual actuation of each segment 610A, 610B, 610C and 610D, as needed. This may allow a single piezoelectric actuator to provide for flow control through more than one fluid channel, may allow for varying degrees of opening/closing of a fluid channel, and the like. In some examples, some of the segments 610A, 610B, 610C and 610D could be selectively actuated to provide for flow control, and some of the segments 610A, 610B, 610C and 610D could be used for sensing (i.e., sensing of flow rates, sensing of pressure and the like). The example arrangement shown in FIG. 6 includes four symmetrically arranged segments 610A, 610B, 610C and 610D that are substantially equal in size and/or shape, simply for purposes of discussion and illustration. The principles to be described herein can be applied to piezoelectric rings having more, or fewer segments having different shapes and/or combinations of shapes and/or different arrangements of segments.

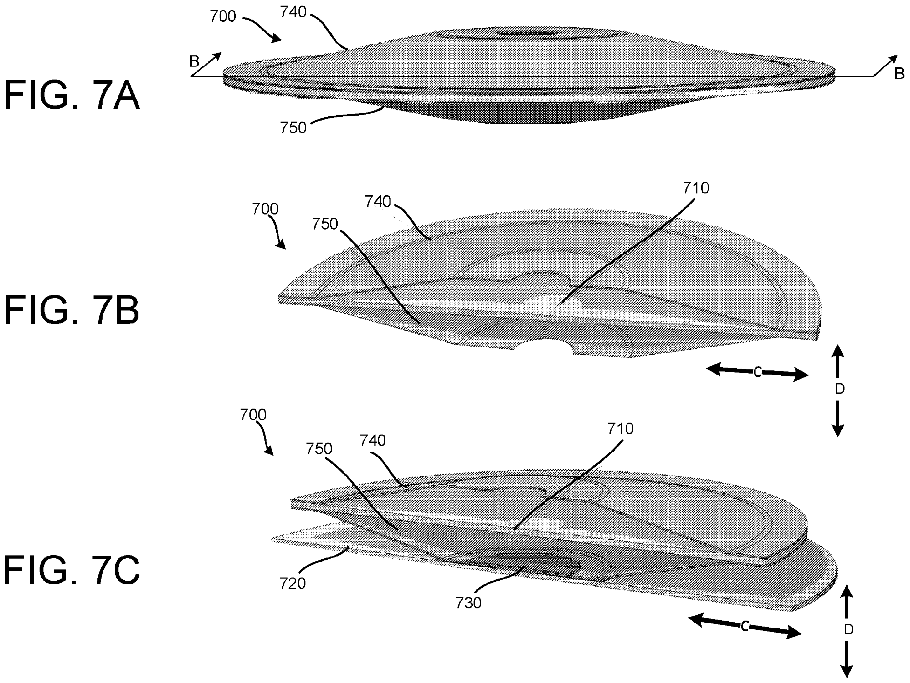

FIG. 7A is a side view, and FIGs. 7B and 7C are a cross-sectional views of an example piezoelectric actuator 700, according to an aspect. The example piezoelectric actuator 700 shown in FIGs. 7A and 7B may be used in a valve and/or a pump of the fluidic architecture of an implantable fluid operated inflatable device according to an aspect.

The example piezoelectric actuator 700 is a cymbal actuator including a piezoelectric element 710 positioned between a first plate 740 and a second plate 750. In some examples, the piezoelectric element 710 has a single layer configuration. In some examples, the piezoelectric element 710 has a multi-layer configuration. One or both of the first plate 740 and the second plate 750 have a convex, or bowl-shaped contour that extends outward from the respective side of the piezoelectric element 710 to which it is coupled. In the example shown in FIGs. 7A-7C, a distance between a central portion of the first plate 740 and the piezoelectric element 710 is greater than a distance between peripheral portions of the first plate 740 and the piezoelectric element 710. Similarly, a distance between a central portion of the second plate 750 and the piezoelectric element 710 is greater than a distance between peripheral portions of the second plate 750 and the piezoelectric element 710. One or both of the first plate 740 and the second plate 750 may be made of a deformable, for example, elastically deformable material. In some examples, one or both of the first plate 740 and the second plate 750 are made of a metal material.

Peripheral edge portions of the piezoelectric element 710 are coupled between corresponding peripheral edge portions of the first and second plates 740, 750. The piezoelectric element 710 can have a variety of different shapes, including for example, a disc shape, a square shape, and other such shapes that allow for peripheral edge portions of the piezoelectric element to be sandwiched between and coupled to the corresponding peripheral edge portions of the first and second plates 740, 750. The piezoelectric actuator 700 may be bonded onto a diaphragm 720 by, for example, an epoxy layer 730, as shown in FIG. 7C. Application of a voltage to the piezoelectric element 710 causes the piezoelectric element 710 to expand or contract in a planar direction corresponding to a plane of the piezoelectric element (for example, horizontally in the example orientation shown in FIGs. 7A-7E, in the direction represented by the arrow(s) C shown in FIGs. 7B and 7C. This planar expansion of the piezoelectric element 710 in turn causes the central portions of the first and second plates 740, 750 to move vertically (in the example orientation shown in FIGs. 7A-7E), for example, the direction represented by the arrow(s) D shown in FIGs. 7B and 7C.

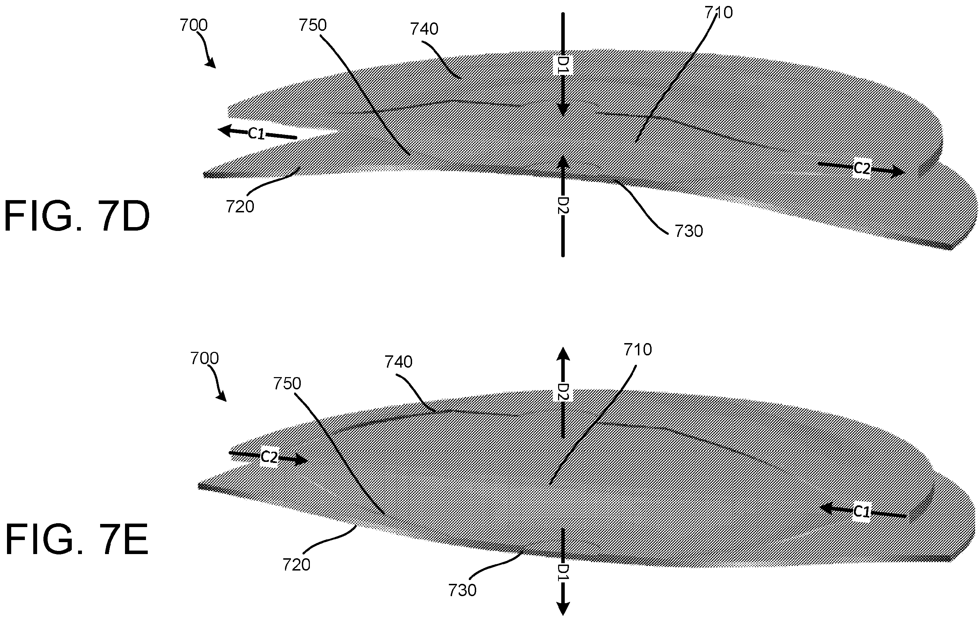

For example, as shown in FIG. 7D, expansion of the piezoelectric element 710 causes a first end portion of the piezoelectric element 710 to expand in the direction of the arrow C1, and a second end portion of the piezoelectric element 710 to expand in the direction of the arrow C2. Due to the coupling of the peripheral edge portions of the piezoelectric element 710 and the first and second plates 740, 750, expansion of the piezoelectric element 710 causes the respective central portions of the first and second plates 740, 750 to be drawn together. That is, expansion of the piezoelectric element 710 causes the corresponding peripheral edge portions of the first and second plates 740, 750 coupled thereto to move outward. Outward movement of the peripheral edge portions of the first and second plates 740, 750 causes the central portion of the first plate 740 to move in the direction of the arrow D1, and the central portion of the second plate 750 to move in the direction of the arrow D2, thus drawing the central portions of the first and second plates 740, 750 closer together. This vertical displacement of the piezoelectric actuator 700 in turn causes displacement, or deformation, or deflection of the diaphragm 720 in the direction of the arrow D2, as shown in FIG. 7D.

Similarly, as shown in FIG. 7E, contraction of the piezoelectric element 710 causes the first end portion of the piezoelectric element 710 to contract in the direction of the arrow C2, and a second end portion of the piezoelectric element 710 to contract in the direction of the arrow C1. Due to the coupling of the peripheral edge portions of the piezoelectric element 710 and the first and second plates 740, 750, contraction of the piezoelectric element 710 causes the respective central portions of the first and second plates 740, 750 move apart. That is, contraction of the piezoelectric element 710 causes the corresponding peripheral edge portions of the first and second plates 740, 750 coupled thereto to move inward. Inward movement of the peripheral edge portions of the first and second plates 740, 750 causes the central portion of the first plate 740 to move in the direction of the arrow D2, and the central portion of the second plate 750 to move in the direction of the arrow D1, thus moving the central portions of the first and second plates 740, 750 further apart. This vertical displacement of the piezoelectric actuator 700 in turn causes displacement, or deformation, or deflection of the diaphragm 720 in the direction of the arrow D1, as shown in FIG. 7E.

**In** the example piezoelectric actuator 700 shown in FIGs. 7A-7E, the piezoelectric element 710 may experience some tensile stress in the expanded condition shown in FIG. 7D, and some compressive stress in the contracted condition shown in FIG. 7E. However, the piezoelectric element 710 is not subjected to bending stresses as voltage is applied, as the piezoelectric element 710 is not directly coupled to the diaphragm 720. Avoidance of the bending stresses that would otherwise be experienced during bending/deformation/deflection of the diaphragm 720 may improve Further, in some examples, the first and second plates 740, 750 may act as a displacement amplifier. Thus, an amount of expansion or contraction of the piezoelectric element 710 that is required to achieve a desired amount of deformation or deflection of the diaphragm 720 may be reduced due to the configuration of the first and/or second plates 740, 750, thus reducing an amount of tensile and/or compressive forces experienced by the piezoelectric element 710. This may further improve the performance and/or durability and/or reliability of the piezoelectric element 710, and of the pump or valve in which the piezoelectric actuator 700 is installed.

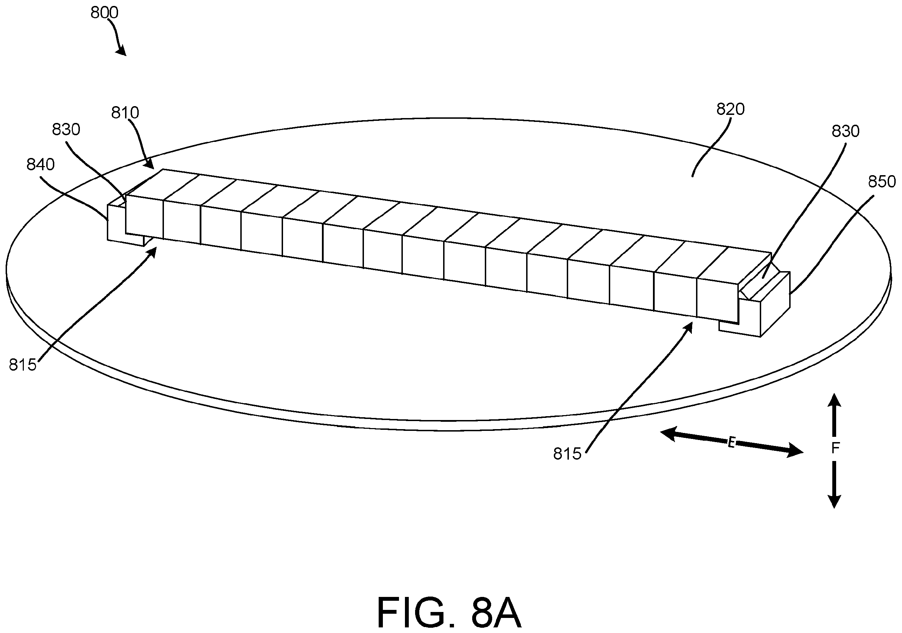

FIG. 8A is a top perspective view of an example piezoelectric actuator 800, according to an aspect. The example piezoelectric actuator 800 shown in FIG. 8A may be used in a valve and/or a pump of the fluidic architecture of an implantable fluid operated inflatable device according to an aspect.

As shown in FIG. 8A, the piezoelectric actuator 800 includes a multilayered piezoelectric element 810 mounted on the diaphragm 820. The multilayered piezoelectric element 810 includes a stack of piezoelectric layers arranged sequentially, from a first end portion to a second end portion of the multilayered piezoelectric element 810. In the example arrangement shown in FIG. 8A, the multilayered piezoelectric element 810 is mounted on the diaphragm 820 by a first support bracket 840 at a first end portion of the piezoelectric element 810, and a second support bracket 850 at a second end portion of the piezoelectric element 810. In some examples, the first and second end portions of the piezoelectric element 810 may be respectively coupled to the first and second support brackets 840, 850 by an epoxy layer 830. The example multilayered piezoelectric element 810 shown in FIG. 8A includes a plurality of substantially rectangular piezoelectric layers arranged in a stack extending diametrically across the diaphragm 820, simply for purposes of discussion and illustration. The principles to be described herein may be applied to a multilayered piezoelectric element including more, or fewer piezoelectric layers and/or a stack of piezoelectric layers having different shapes and/or a stack of piezoelectric layers arranged differently than shown in FIG. 8A.

The first and second end portions of the multilayered piezoelectric element 810 are coupled between the first and second support brackets 840, 850 mounted on the diaphragm 820. In the example arrangement shown in FIG. 8A, the stack of piezoelectric layers forming the multilayered piezoelectric element 810 is coupled to the first and second support brackets 840, 850, but does not directly contact the diaphragm 820. Rather, the portion of the multilayered piezoelectric element 810 between the first and second end portions thereof is spaced apart from the mounting surface of the diaphragm 820, such that a space 815 is formed between a bottom portion of the multilayered piezoelectric element 810 and the mounting surface of the diaphragm 820. Thus, the intermediate portion of the multilayered piezoelectric element 810, between the first end portion and the second end portion thereof, is movable relative to the diaphragm 820, and not fixed to the diaphragm 820. Application of a voltage to the piezoelectric element 810 causes the piezoelectric element 810 to expand or contract horizontally (in the example orientation shown in FIG. 8A), for example, the direction represented by the arrow(s) E shown in FIG. 8A, with corresponding movement of the first and second support brackets 840, 850, and corresponding vertical displacement of the piezoelectric element 810, the first and second support brackets 840, 850 and the diaphragm 820 (in the example orientation shown in 8A), for example, the direction represented by the arrow(s) F shown in FIG. 8A.

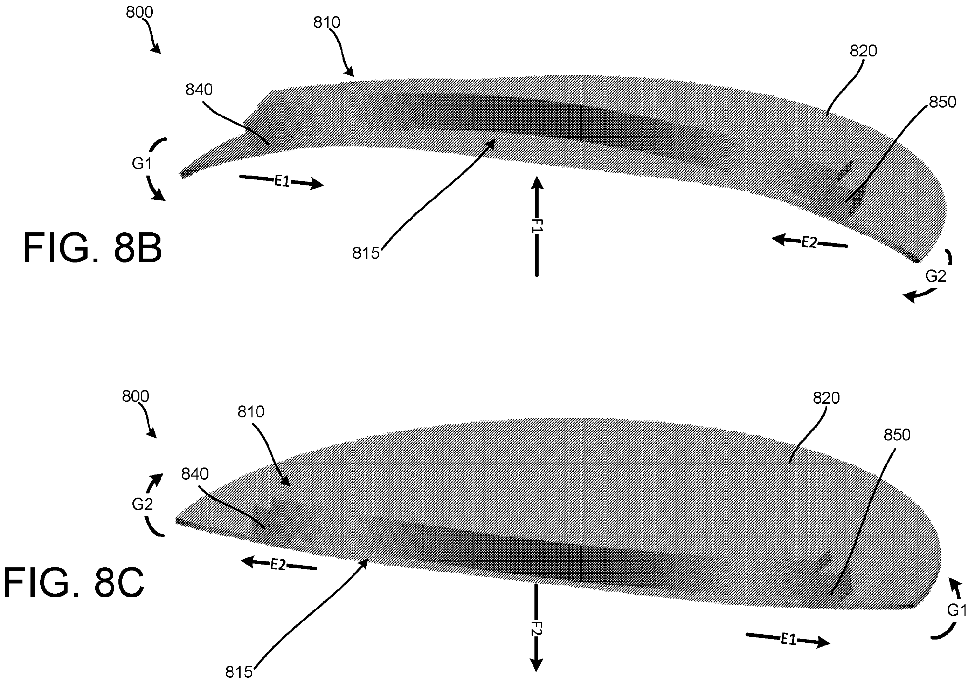

For example, as shown in FIG. 8B, expansion of the piezoelectric element 810 in the direction of the arrows E1 and E2, while the first and second support brackets 840, 850 remain fixed between the piezoelectric element 810 and the diaphragm 820 causes pivoting in the direction of the arrow G1 at the first support bracket 840/first end portion of the piezoelectric element 810, and pivoting in the direction of the arrow G2 at the second support bracket 850/second end portion of the piezoelectric element 810. Thus, in this arrangement, the first support bracket 840 acts as a pivot allowing the first end portion of the piezoelectric element 810 to pivot in the direction of the arrow G1, and the second support bracket 850 acts as a pivot allowing the second end portion of the piezoelectric element 810 to pivot in the direction of the arrow G2. The coupling of the first and second support brackets 840, 850 between the piezoelectric element 810 and the diaphragm 820 as the applied voltage causes the piezoelectric element 810 to expand in this manner causes a bending, or deformation, or deflection of the diaphragm 820 in the direction of the arrow F1, as shown in FIG. 8B.

Similarly, as shown in FIG. 8C, contraction of the piezoelectric element 810 in the direction of the arrow E2 at the first end portion thereof and the arrow E1 at the second end portion thereof, while the first and second support brackets 840, 850 remain fixed between the piezoelectric element 810 and the diaphragm 820 causes pivoting in the direction of the arrow G2 at the first support bracket 840/first end portion of the piezoelectric element 810, and pivoting in the direction of the arrow G1 at the second support bracket 850/second end portion of the piezoelectric element 810. In this arrangement, the first support bracket 840 acts as a pivot allowing the first end portion of the piezoelectric element 810 to pivot in the direction of the arrow G2, and the second support bracket 850 acts as a pivot allowing the second end portion of the piezoelectric element 810 to pivot in the direction of the arrow G1. The coupling of the first and second support brackets 840, 850 between the piezoelectric element 810 and the diaphragm 820 as the applied voltage causes the piezoelectric element 810 to contract in this manner causes a bending, or deformation, or deflection of the diaphragm 820 in the direction of the arrow F2, as shown in FIG. 8C.

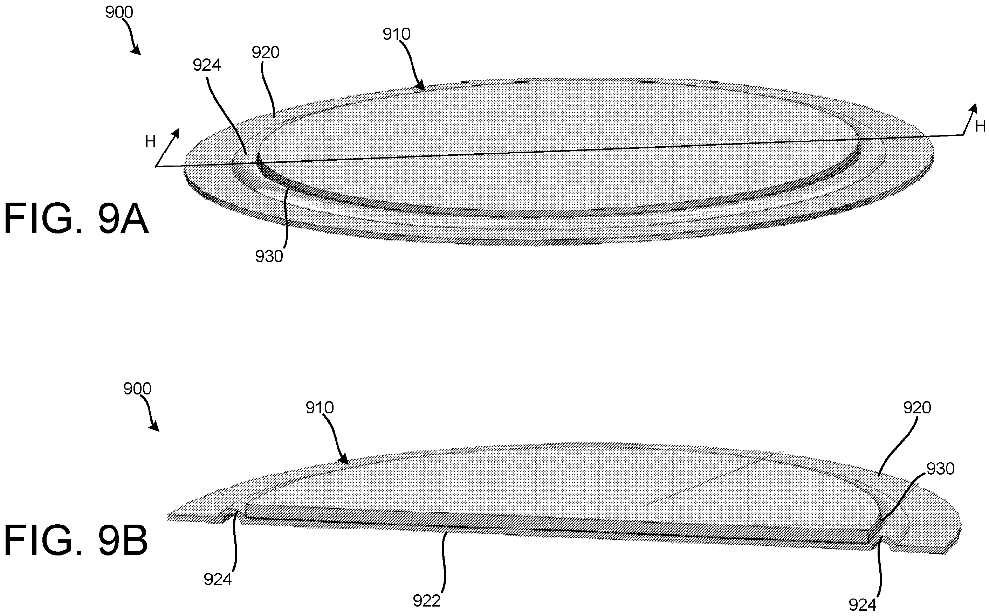

FIG. 9A is a top perspective view, and FIG. 9B is a cross sectional view, of an example piezoelectric actuator 900, according to an aspect. The example piezoelectric actuator 900 shown in FIGs. 9A and 9B may be used in a valve and/or a pump of the fluidic architecture of an implantable fluid operated inflatable device according to an aspect.

As shown in FIGs. 9A and 9B, the piezoelectric actuator 900 includes a piezoelectric element 910 mounted on a diaphragm 920. In some examples, an adhesive layer 930, or an epoxy layer 930, is disposed between the piezoelectric element 910 and the diaphragm 920 to couple the piezoelectric element 910 on the diaphragm 920. In the example arrangement shown in FIGs. 9A and 9B, the piezoelectric element 910 is substantially circular, and in the form of a disc, simply for purposes of discussion and illustration. Similarly, the diaphragm 920 shown in FIGs. 9A and 9B is substantially circular, simply for purposes of discussion and illustration. The principles to be described herein can be applied to piezoelectric elements and/or diaphragms having other shapes and/or configurations.

In the example arrangement shown in FIGs. 9A and 9B, the piezoelectric element 910 is coupled on a flat portion 922 of the diaphragm 920, within a protrusion 924, or corrugation 924, surrounding the flat portion 922. Thus, the piezoelectric element 910 is received within an area of the diaphragm 920 that is enclosed within the protrusion 924. The protrusion 924 surrounding the flat portion 922 of the diaphragm 920 may allow for a greater amount of displacement, or deflection of the diaphragm 920 for a given level of voltage applied to the piezoelectric element 910. This may improve the performance and/or reliability of the piezoelectric actuator 900 and/or the pump or valve in which the piezoelectric actuator 900 is installed. Additionally, the protrusion 924 surrounding the flat portion 922 of the diaphragm 920 may improve alignment of the piezoelectric element 910 on the diaphragm 920 during fabrication, thus reducing variation between piezoelectric actuators 900 configured in this manner, and further improving performance and/or reliability of the piezoelectric actuator 900 and/or the pump or valve in which the piezoelectric actuator 900 is installed.

## Claims

1. An implantable fluid operated inflatable device (100; 100A; 100B), comprising:
a fluid reservoir (102; 102A; 102B);
an inflatable member (104; 104A; 104B); and
a fluid control system (106; 106A; 106B) configured to control fluid flow between the fluid reservoir (102; 102A; 102B) and the inflatable member (104; 104A; 104B), the fluid control system (106; 106A; 106B) including:
a housing (110A; 110B);
fluidic architecture defining one or more fluid passageways within in the housing (110A; 110B); and
at least one pump and at least one valve (400) positioned in the one or more fluid passageways, the at least one pump and the at least one valve (400) including a piezoelectric actuator (500; 600; 700; 800; 900) that is operable in response to a voltage applied thereto by an electronic control system (108; 108A; 108B) of the fluid control system (106; 106A; 106B), the piezoelectric actuator (500; 600; 700; 800; 900) including:
a diaphragm (520; 620; 720; 820; 920); and
a piezoelectric element (510; 610; 710; 810; 910) coupled to the diaphragm (520; 620; 720; 820; 920) and configured to deform in response to a voltage applied thereto by the electronic control (108; 108A; 108B) system,
wherein the diaphragm (520; 620; 720; 820; 920) is configured to deform in response to deformation of the piezoelectric element (510; 610; 710; 810; 910) mounted thereon, and
wherein the piezoelectric actuator (500; 600; 700; 800; 900) actuates the at least one pump and the at least one valve (400) to control a flow of fluid in the one or more fluid passageways based on one of an amount of the deformation of the diaphragm (520; 620; 720; 820; 920) or a direction of the deformation of the diaphragm (520; 620; 720; 820; 920).

2. The implantable fluid operated inflatable device (100; 100A; 100B) of claim 1, wherein the piezoelectric element (510; 610) includes:
a plate portion (514; 614) mounted on the diaphragm (520; 620); and
an open portion (512; 612) in the plate portion (514; 614) such that the plate portion (514; 614) surrounds the open portion (512; 612), wherein a position of the open portion (512; 612) of the piezoelectric element (510; 610) corresponds to a high bending stress region of the piezoelectric actuator (500; 600) during deformation of the piezoelectric actuator (500; 600).

3. The implantable fluid operated inflatable device (100; 100A; 100B) of claim 1 or claim 2, wherein the open portion (512; 612) of the piezoelectric element (510; 610) is defined by a substantially circular opening at a central portion of the piezoelectric element (510; 610), and the plate portion (514; 614) forms an annular ring surrounding the open portion (512; 612).

4. The implantable fluid operated inflatable device (100; 100A; 100B) of any preceding claim, wherein the plate portion (614) includes a plurality of separate segments (610A, 610B, 610C, 610D) arranged surrounding the opening, wherein each of the plurality of separate segments (610A, 610B, 610C, 610D) is separately actuatable in response to a voltage applied thereto by the electronic control system (108; 108A; 108B) of the fluid control system (106; 106A; 106B).

5. The implantable fluid operated inflatable device (100; 100A; 100B) of claim 4, wherein a size and a shape of each of the plurality of separate segments (610A, 610B, 610C, 610D) is substantially the same, and wherein the plurality of separate segments (610A, 610B, 610C, 610D) is arranged substantially symmetrically about a central plane of the piezoelectric element (610).

6. The implantable fluid operated inflatable device (100; 100A; 100B) of any preceding claim, further comprising a protrusion (924) extending along a peripheral portion of the diaphragm (920), wherein a contour of the protrusion (924) corresponds to an outer peripheral contour of the plate portion of the piezoelectric element (910) such that the piezoelectric element (910) is received within an area of the diaphragm (920) enclosed by the protrusion (924).

7. The implantable fluid operated inflatable device (100; 100A; 100B) of claim 1, wherein the piezoelectric actuator (700) includes:
a piezoelectric element (710) configured to deform in response to a voltage applied thereto by an electronic control system (108) of the fluid control system (106);
a first plate (740) coupled to a first side of the piezoelectric element (710), the first plate (740) having a convex contour relative to the first side of the piezoelectric element (710);
a second plate (750) coupled to a second side of the piezoelectric element (710), the second side being opposite the first side of the piezoelectric element (710), the second plate (750) having a convex contour relative to the second side of the piezoelectric element (710); and
a diaphragm (720) coupled to the second plate (750), wherein the diaphragm (720) is configured to deform in response to deformation of the piezoelectric element (710).

8. The implantable fluid operated inflatable device (100; 100A; 100B) of claim 7, wherein:
a distance between a central portion of the first plate (740) and the diaphragm (720) is greater than a distance between peripheral portions of the first plate (740) and the diaphragm (720); and
a distance between a central portion of the second plate (750) and the diaphragm (720) is greater than a distance between peripheral portions of the second plate (750) and the diaphragm (720).

9. The implantable fluid operated inflatable device (100; 100A; 100B) of claim 7 or claim 8, wherein:
peripheral edge portions of the first plate (740) are coupled to peripheral edge portions of the first side of the piezoelectric element (710);
peripheral edge portions of the second plate (750) are coupled to peripheral edge portions of the second side of the piezoelectric element (710); and
the diaphragm (720) is coupled to a central portion of a central portion of the second plate (750), with the second plate (750) positioned between the diaphragm (720) and the piezoelectric element (710).

10. The implantable fluid operated inflatable device (100; 100A; 100B) of any of claims 7 through 9, wherein:
the piezoelectric element (710) is configured to expand in a planar direction in response to a first voltage applied thereto, and to contract in the planar direction in response to a second voltage applied thereto; and
the first plate (740) and the second plate (750) are configured to deform in response to expansion or contraction of the piezoelectric element (710).

11. The implantable fluid operated inflatable device (100; 100A; 100B) of claim 10, wherein:
the first plate (740) is configured to deform in a first direction and the second plate (750) is configured to deform in a second direction opposite the first direction in response to expansion of the piezoelectric element (710) to decrease a distance between the first plate (740) and the second plate (750);
the first plate (740) is configured to deform in the second direction and the second plate (750) is configured to deform in the first direction in response to contraction of the piezoelectric element (710) to increase a distance between the first plate (740) and the second plate (750);
the diaphragm (720) is configured to deform in the first direction in response to expansion of the piezoelectric element (710); and
the diaphragm (720) is configured to deform in the second direction in response to contraction of the piezoelectric element (710).

12. The implantable fluid operated inflatable device (100., 100A; 100B) of claim 1, wherein the piezoelectric element (810) includes:
a stack of piezoelectric layers, including a plurality of piezoelectric layers arranged sequentially along a mounting surface of the diaphragm (820), from a first end portion to a second end portion of the stack of piezoelectric layers;
a first support bracket (840) coupling a first piezoelectric layer, at the first end portion of the stack of piezoelectric layers, to a mounting surface of the diaphragm (820); and
a second support bracket (850) coupling a second piezoelectric layer, at the second end portion of the stack of piezoelectric layers, to the mounting surface of the diaphragm (820).

13. The implantable fluid operated inflatable device (100; 100A; 100B) of claim 12, wherein:
the stack of piezoelectric layers is configured to expand in response to a first voltage applied thereto;
the first end portion of the stack of piezoelectric layers is configured to pivot in a first pivotal direction at the first support bracket (840) in response to expansion of the stack of piezoelectric layers; and
the second end portion of the stack of piezoelectric layers is configured to pivot in a second pivotal direction at the second support bracket (850) in response to expansion of the stack of piezoelectric layers.

14. The implantable fluid operated inflatable device (100) of claim 12 or claim 13, wherein:
the stack of piezoelectric layers is configured to contract in response to a second voltage applied thereto;
the first end portion of the stack of piezoelectric layers is configured to pivot in the second pivotal direction at the first support bracket (840) in response to contraction of the stack of piezoelectric layers; and
the second end portion of the stack of piezoelectric layers is configured to pivot in the first pivotal direction at the second support bracket (850) in response to contraction of the stack of piezoelectric layers.

15. The implantable fluid operated inflatable device (100; 100A; 100B) of any of claims 12 through 14, wherein:
the diaphragm (820) is configured to deform in a first direction in response to expansion of the stack of piezoelectric layers; and
the diaphragm (820) is configured to deform in a second direction in response to contraction of the stack of piezoelectric layers, and wherein a space (815) is formed between a bottom portion of the piezoelectric element (810) and the mounting surface of the diaphragm (820), and wherein a distance between the bottom portion of the piezoelectric element (810) and the mounting surface of the diaphragm (820) increases in response to expansion of the stack of piezoelectric layers, and decreases in response to contraction of the stack of piezoelectric layers.

## Patentansprüche

1. Implantierbare fluidbetriebene fluidexpandierbare Vorrichtung (100; 100A; 100B), umfassend:
ein Fluidreservoir (102; 102A; 102B);
ein fluidexpandierbares Element (104; 104A; 104B); und
ein Fluidsteuerungssystem (106; 106A; 106B), das eingerichtet ist, den Fluidstrom zwischen dem Fluidreservoir (102; 102A; 102B) und dem fluidexpandierbaren Element (104; 104A; 104B) zu steuern, wobei das Fluidsteuerungssystem (106; 106A; 106B) enthält:
ein Gehäuse (110A; HOB);
eine Fluidarchitektur, die einen oder mehrere Fluidkanäle innerhalb des Gehäuses (110A; HOB) definiert; und
mindestens eine Pumpe und mindestens ein Ventil (400), die in dem einen oder den mehreren Fluidkanälen angeordnet sind, wobei die mindestens eine Pumpe und das mindestens eine Ventil (400) einen piezoelektrischen Aktuator (500; 600; 700; 800; 900) umfassen, der als Reaktion auf eine durch ein elektronisches Steuerungssystem (108; 108A; 108B) des Fluidsteuerungssystems (106; 106A; 106B) angelegte Spannung betätigbar ist, wobei der piezoelektrische Aktuator (500; 600; 700; 800; 900) umfasst:
eine Membran (520; 620; 720; 820; 920); und
ein piezoelektrisches Element (510; 610; 710; 810; 910), das mit der Membran (520; 620; 720; 820; 920) gekoppelt ist und eingerichtet ist, sich als Reaktion auf eine durch das elektronische Steuerungssystem (108; 108A; 108B) angelegte Spannung zu verformen,
wobei die Membran (520; 620; 720; 820; 920) eingerichtet ist, sich als Reaktion auf eine Verformung des darauf montierten piezoelektrischen Elements (510; 610; 710; 810; 910) zu verformen, und
wobei der piezoelektrische Aktuator (500; 600; 700; 800; 900) die mindestens eine Pumpe und das mindestens eine Ventil (400) betätigt, um einen Fluidstrom in dem einen oder den mehreren Fluidkanälen auf der Grundlage entweder eines Ausmaßes der Verformung der Membran (520; 620; 720; 820; 920) oder einer Richtung der Verformung der Membran (520; 620; 720; 820; 920) zu steuern.

2. Implantierbare fluidbetriebene fluidexpandierbare Vorrichtung (100; 100A; 100B) nach Anspruch 1, wobei das piezoelektrische Element (510; 610) umfasst:
einen an der Membran (520; 620) montierten Plattenteil (514; 614); und
einen offenen Teil (512; 612) in dem Plattenteil (514; 614), so dass der Plattenteil (514; 614) den offenen Teil (512; 612) umgibt, wobei eine Position des offenen Teils (512; 612) des piezoelektrischen Elements (510; 610) einem Bereich hoher Biegespannung des piezoelektrischen Aktuators (500; 600) während der Verformung des piezoelektrischen Aktuators (500; 600) entspricht.

3. Implantierbare fluidbetriebene fluidexpandierbare Vorrichtung (100; 100A; 100B) nach Anspruch 1 oder Anspruch 2, wobei der offene Teil (512; 612) des piezoelektrischen Elements (510; 610) durch eine im Wesentlichen kreisförmige Öffnung in einem mittigen Teil des piezoelektrischen Elements (510; 610) definiert ist und der Plattenteilt (514; 614) einen den offenen Teil (512; 612) umgebenden Ring bildet.

4. Implantierbare fluidbetriebene fluidexpandierbare Vorrichtung (100; 100A; 100B) nach einem der vohergehenden Ansprüchen, wobei der Plattenteil (614) eine Vielzahl separater Segmente (610A, 610B, 610C, 610D) umfasst, die die Öffnung umgebend angeordnet sind, wobei jedes der Vielzahl separater Segmente (610A, 610B, 610C, 610D) separat in Reaktion auf eine durch das elektronische Steuerungssystem (108; 108A; 108B) des Fluidsteuerungssystems daran angelegte Spannung betätigbar ist.

5. Implantierbare fluidbetriebene fluidexpandierbare Vorrichtung (100; 100A; 100B) nach Anspruch 4, wobei eine Größe und eine Form jedes der mehreren einzelnen Segmente (610A, 610B, 610C, 610D) im Wesentlichen gleich ist und wobei die Vielzahl separater Segmente (610A, 610B, 610C, 610D) im Wesentlichen symmetrisch um eine Mittelebene des piezoelektrischen Elements (610) angeordnet ist.

6. Implantierbare fluidbetriebene fluidexpandierbare Vorrichtung (100; 100A; 100B) nach einem der vohergehenden Ansprüchen, die ferner einen Vorsprung (924) aufweist, der sich entlang eines Umfangbereichs der Membran (920) erstreckt, wobei eine Kontur des Vorsprungs (924) einer äußeren Umfangskontur des Plattenteils des piezoelektrischen Elements (910) entspricht, so dass das piezoelektrische Element (910) innerhalb eines Bereichs der Membran (920) aufgenommen wird, der von dem Vorsprung (924) umschlossen ist.

7. Implantierbare fluidbetriebene fluidexpandierbare Vorrichtung (100; 100A; 100B) nach Anspruch 1, wobei der piezoelektrische Aktuator (700) umfasst:
ein piezoelektrisches Element (710), das eingerichtet ist, sich als Reaktion auf eine durch ein elektronisches Steuerungssystem (108) des Fluidsteuerungssystems (106) daran angelegte Spannung zu verformen;
eine erste Platte (740), die mit einer ersten Seite des piezoelektrischen Elements (710) gekoppelt ist, wobei die erste Platte (740) im Verhältnis zur ersten Seite des piezoelektrischen Elements (710) eine konvexe Kontur aufweist;
eine zweite Platte (750), die mit einer zweiten Seite des piezoelektrischen Elements (710) verbunden ist, wobei die zweite Seite der ersten Seite des piezoelektrischen Elements (710) gegenüberliegt und die zweite Platte (750) im Verhältnis zur zweiten Seite des piezoelektrischen Elements (710) eine konvexe Kontur aufweist; und
eine Membran (720), die mit der zweiten Platte (750) gekoppelt ist, wobei die Membran (720) eingerichtet ist, sich als Reaktion auf die Verformung des piezoelektrischen Elements (710) zu verformen.

8. Implantierbare fluidbetriebene fluidexpandierbare Vorrichtung (100; 100A; 100B) nach Anspruch 7, wobei:
ein Abstand zwischen einem mittleren Teil der ersten Platte (740) und der Membran (720) größer ist als ein Abstand zwischen Randbereichen der ersten Platte (740) und der Membran (720); und
ein Abstand zwischen einem mittleren Teil der zweiten Platte (750) und der Membran (720) größer ist als ein Abstand zwischen Randbereichen der zweiten Platte (750) und der Membran (720).

9. Implantierbare fluidbetriebene fluidexpandierbare Vorrichtung (100; 100A; 100B) nach Anspruch 7 oder 8, wobei:
Randbereiche der ersten Platte (740) mit Randbereichen der ersten Seite des piezoelektrischen Elements (710) gekoppelt sind;
Randbereiche der zweiten Platte (750) mit Randbereichen der zweiten Seite des piezoelektrischen Elements (710) gekoppelt sind; und
die Membran (720) mit einem mittleren Teil eines mittleren Teils der zweiten Platte (750) gekoppelt ist, wobei die zweite Platte (750) zwischen der Membran (720) und dem piezoelektrischen Element (710) angeordnet ist.

10. Implantierbare fluidbetriebene fluidexpandierbare Vorrichtung (100; 100A; 100B) nach einem der Ansprüche 7 bis 9, wobei:
das piezoelektrische Element (710) eingerichtet ist, sich in einer ebenen Richtung auszudehnen, wenn eine erste Spannung daran angelegt wird, und sich in der ebenen Richtung zusammenzuziehen, wenn eine zweite Spannung daran angelegt wird; und
die erste Platte (740) und die zweite Platte (750) eingerichtet sind, sich als Reaktion auf die Ausdehnung oder Kontraktion des piezoelektrischen Elements (710) zu verformen.

11. Implantierbare fluidbetriebene fluidexpandierbare Vorrichtung (100; 100A; 100B) nach Anspruch 10, wobei:
die erste Platte (740) eingerichtet ist, sich in einer ersten Richtung zu verformen, und die zweite Platte (750) eingerichtet ist, sich in einer zweiten Richtung, die der ersten Richtung entgegengesetzt ist, zu verformen, als Reaktion auf eine Ausdehnung des piezoelektrischen Elements (710), um einen Abstand zwischen der ersten Platte (740) und der zweiten Platte (750) zu verringern;
die erste Platte (740) eingerichtet ist, sich in der zweiten Richtung zu verformen, und die zweite Platte (750) eingerichtet ist, sich in der ersten Richtung zu verformen, als Reaktion auf eine Kontraktion des piezoelektrischen Elements (710), um einen Abstand zwischen der ersten Platte (740) und der zweiten Platte (750) zu vergrößern;
die Membran (720) eingerichtet ist, sich als Reaktion auf eine Ausdehnung des piezoelektrischen Elements (710) in die erste Richtung zu verformen; und
die Membran (720) eingerichtet ist, sich als Reaktion auf eine Kontraktion des piezoelektrischen Elements (710) in die zweite Richtung zu verformen.

12. Implantierbare fluidbetriebene fluidexpandierbare Vorrichtung (100; 100A; 100B) nach Anspruch 1, wobei das piezoelektrische Element (810) umfasst:
einen Stapel piezoelektrischer Schichten, der eine Vielzahl von piezoelektrischen Schichten umfasst, die nacheinander entlang einer Montagefläche der Membran (820) angeordnet sind, und zwar von einem ersten Endteil bis zu einem zweiten Endteil des Stapels piezoelektrischer Schichten;
eine erste Halterung (840), die eine erste piezoelektrische Schicht am ersten Endteil des Stapels piezoelektrischer Schichten mit einer Montagefläche der Membran (820) koppelt; und
eine zweite Halterung (850), die eine zweite piezoelektrische Schicht am zweiten Endteil des Stapels aus piezoelektrischen Schichten mit der Montagefläche der Membran (820) koppelt.

13. Implantierbare fluidbetriebene fluidexpandierbare Vorrichtung (100; 100A; 100B) nach Anspruch 12, wobei:
der Stapel aus piezoelektrischen Schichten eingerichtet ist, sich in Reaktion auf eine daran angelegte erste Spannung auszudehnen;
der vordere Endteil des Stapels piezoelektrischer Schichten eingerichtet ist, an der ersten Halterung (840) als Reaktion auf eine Ausdehnung des Stapels piezoelektrischer Schichten in eine erste Schwenkrichtung zu schwenken; und
der zweite Endteil des Stapels piezoelektrischer Schichten eingerichtet ist, an der zweiten Halterung (850) in Reaktion auf eine Ausdehnung des Stapels piezoelektrischer Schichten in eine zweite Schwenkrichtung zu schwenken.

14. Implantierbare fluidbetriebene fluidexpandierbare Vorrichtung (100) nach Anspruch 12 oder Anspruch 13, wobei:
der Stapel aus piezoelektrischen Schichten eingerichtet ist, sich in Reaktion auf eine daran angelegte erste Spannung auszudehnen;
der erste Endteil des Stapels piezoelektrischer Schichten eingerichtet ist, an der ersten Halterung (840) in Reaktion auf eine Kontraktion des Stapels piezoelektrischer Schichten in die zweite Schwenkrichtung zu schwenken; und
der zweite Endteil des Stapels piezoelektrischer Schichten eingerichtet ist, an der zweiten Halterung (850) in Reaktion auf eine Kontraktion des Stapels piezoelektrischer Schichten in die erste Schwenkrichtung zu schwenken.

15. Implantierbare fluidbetriebene fluidexpandierbare Vorrichtung (100; 100A; 100B) nach einem der Ansprüche 12 bis 14, wobei:
die Membran (820) eingerichtet ist, sich in Reaktion auf eine Ausdehnung des piezoelektrischen Elements in die erste Richtung zu verformen; und
die Membran (820) eingerichtet ist, sich in Reaktion auf eine Kontraktion des Stapels piezoelektrischer Schichten in eine zweite Richtung zu verformen, und wobei zwischen einem unteren Teil des piezoelektrischen Elements (810) und der Montagefläche der Membran (820) ein Raum (815) gebildet ist, und wobei sich ein Abstand zwischen dem unteren Teil des piezoelektrischen Elements (810) und der Montagefläche der Membran (820) in reaktion auf eine Ausdehnung des Stapels aus piezoelektrischen Schichten vergrößert und in Reaktion auf eine Kontraktion des Stapels aus piezoelektrischen Schichten verringert.

## Revendications

1. Dispositif gonflable piloté par un fluide et implantable (100 ; 100A ; 100B), comprenant :
un réservoir de fluide (102 ; 102A ; 102B) ;
un élément gonflable (104; 104A; 104B) ; et
un système de commande de fluide (106; 106A; 106B) configuré pour commander un écoulement de fluide entre le réservoir de fluide (102 ; 102A ; 102B) et l'élément gonflable (104; 104A ; 104B), le système de commande de fluide (106 ; 106A ; 106B) incluant :
un boîtier (110A ; 110B) ;
une architecture fluidique qui définit un ou plusieurs passages de fluide à l'intérieur du boîtier (110A; 110B) ; et
au moins une pompe et au moins une soupape (400) positionnées dans les un ou plusieurs passages de fluide, l'au moins une pompe et l'au moins une soupape (400) incluant un actionneur piézoélectrique (500 ; 600 ; 700 ; 800 ; 900) qui peut être piloté en réponse à une tension qui lui est appliquée par un système de commande électronique (108; 108A; 108B) du système de commande de fluide (106 ; 106A; 106B), l'actionneur piézoélectrique (500 ; 600 ; 700 ; 800 ; 900) incluant :
un diaphragme (520 ; 620 ; 720 ; 820 ; 920) ; et
un élément piézoélectrique (510 ; 610 ; 710 ; 810 ; 910) couplé au diaphragme (520 ; 620 ; 720 ; 820 ; 920) et configuré pour être déformé en réponse à une tension qui lui est appliquée par le système de commande électronique (108 ; 108A ; 108B),
dans lequel le diaphragme (520 ; 620 ; 720 ; 820 ; 920) est configuré pour être déformé en réponse à une déformation de l'élément piézoélectrique (510 ; 610 ; 710 ; 810 ; 910) qui est monté dessus, et
dans lequel l'actionneur piézoélectrique (500 ; 600 ; 700 ; 800 ; 900) actionne l'au moins une pompe et l'au moins une soupape (400) afin de commander un écoulement de fluide dans les un ou plusieurs passages de fluide sur la base d'un paramètre parmi une quantité de la déformation du diaphragme (520 ; 620 ; 720 ; 820 ; 920) et une direction de la déformation du diaphragme (520 ; 620 ; 720 ; 820 ; 920).

2. Dispositif gonflable piloté par un fluide et implantable (100 ; 100A ; 100B) selon la revendication 1, dans lequel l'élément piézoélectrique (510 ; 610) inclut :
une partie de plaque plane (514 ; 614) montée sur le diaphragme (520 ; 620) ; et
une partie ouverte (512 ; 612) dans la partie de plaque plane (514; 614) de telle sorte que la partie de plaque plane (514; 614) entoure la partie ouverte (512 ; 612), dans lequel une position de la partie ouverte (512 ; 612) de l'élément piézoélectrique (510 ; 610) correspond à une région de contrainte de flexion élevée de l'actionneur piézoélectrique (500 ; 600) pendant la déformation de l'actionneur piézoélectrique (500 ; 600).

3. Dispositif gonflable piloté par un fluide et implantable (100 ; 100A ; 100B) selon la revendication 1 ou la revendication 2, dans lequel la partie ouverte (512 ; 612) de l'élément piézoélectrique (510 ; 610) est définie par une ouverture sensiblement circulaire au niveau d'une partie centrale de l'élément piézoélectrique (510 ; 610), et la partie de plaque plane (514 ; 614) forme une bague annulaire qui entoure la partie ouverte (512 ; 612).

4. Dispositif gonflable piloté par un fluide et implantable (100 ; 100A ; 100B) selon l'une quelconque des revendications précédentes, dans lequel la partie de plaque plane (614) inclut une pluralité de segments séparés (610A, 610B, 610C, 610D) qui sont agencés de manière à entourer l'ouverture, dans lequel chacun de la pluralité de segments séparés (610A, 610B, 610C, 610D) peut être actionné séparément en réponse à une tension qui lui est appliquée par le système de commande électronique (108 ; 108A ; 108B) du système de commande de fluide (106; 106A ; 106B).

5. Dispositif gonflable piloté par un fluide et implantable (100 ; 100A ; 100B) selon la revendication 4, dans lequel les dimensions et les formes des segments de la pluralité de segments séparés (610A, 610B, 610C, 610D) sont respectivement sensiblement les mêmes, et dans lequel les segments de la pluralité de segments séparés (610A, 610B, 610C, 610D) sont agencés de façon sensiblement symétrique autour d'un plan central de l'élément piézoélectrique (610).

6. Dispositif gonflable piloté par un fluide et implantable (100 ; 100A ; 100B) selon l'une quelconque des revendications précédentes, comprenant en outre une protubérance (924) qui est étendue le long d'une partie périphérique du diaphragme (920), dans lequel un contour de la protubérance (924) correspond à un contour périphérique externe de la partie de plaque plane de l'élément piézoélectrique (910) de telle sorte que l'élément piézoélectrique (910) soit reçu à l'intérieur d'une zone du diaphragme (920) qui est renfermée par la protubérance (924).

7. Dispositif gonflable piloté par un fluide et implantable (100 ; 100A ; 100B) selon la revendication 1, dans lequel l'actionneur piézoélectrique (700) inclut :
un élément piézoélectrique (710) configuré pour être déformé en réponse à une tension qui lui est appliquée par un système de commande électronique (108) du système de commande de fluide (106) ;
une première plaque (740) couplée à un premier côté de l'élément piézoélectrique (710), la première plaque (740) présentant un contour convexe par rapport au premier côté de l'élément piézoélectrique (710) ;
une seconde plaque (750) couplée à un second côté de l'élément piézoélectrique (710), le second côté étant opposé au premier côté de l'élément piézoélectrique (710), la seconde plaque (750) présentant un contour convexe par rapport au second côté de l'élément piézoélectrique (710) ; et
un diaphragme (720) couplé à la seconde plaque (750), dans lequel le diaphragme (720) est configuré pour être déformé en réponse à la déformation de l'élément piézoélectrique (710).

8. Dispositif gonflable piloté par un fluide et implantable (100 ; 100A ; 100B) selon la revendication 7, dans lequel :
une distance entre une partie centrale de la première plaque (740) et le diaphragme (720) est plus grande qu'une distance entre des parties périphériques de la première plaque (740) et le diaphragme (720) ; et
une distance entre une partie centrale de la seconde plaque (750) et le diaphragme (720) est plus grande qu'une distance entre des parties périphériques de la seconde plaque (750) et le diaphragme (720).

9. Dispositif gonflable piloté par un fluide et implantable (100 ; 100A ; 100B) selon la revendication 7 ou la revendication 8, dans lequel :
des parties de bord périphérique de la première plaque (740) sont couplées à des parties de bord périphérique du premier côté de l'élément piézoélectrique (710) ;
des parties de bord périphérique de la seconde plaque (750) sont couplées à des parties de bord périphérique du second côté de l'élément piézoélectrique (710) ; et
le diaphragme (720) est couplé à une partie centrale d'une partie centrale de la seconde plaque (750), la seconde plaque (750) étant positionnée entre le diaphragme (720) et l'élément piézoélectrique (710).

10. Dispositif gonflable piloté par un fluide et implantable (100 ; 100A ; 100B) selon l'une quelconque des revendications 7 à 9, dans lequel :
l'élément piézoélectrique (710) est configuré pour être dilaté dans une direction plane en réponse à une première tension qui lui est appliquée et pour être contracté dans la direction plane en réponse à une seconde tension qui lui est appliquée ; et
la première plaque (740) et la seconde plaque (750) sont configurées pour être déformées en réponse à une dilatation ou à une contraction de l'élément piézoélectrique (710).

11. Dispositif gonflable piloté par un fluide et implantable (100 ; 100A ; 100B) selon la revendication 10, dans lequel :
la première plaque (740) est configurée pour être déformée dans une première direction et la seconde plaque (750) est configurée pour être déformée dans une seconde direction opposée à la première direction en réponse à une dilatation de l'élément piézoélectrique (710) afin de diminuer une distance entre la première plaque (740) et la seconde plaque (750) ;
la première plaque (740) est configurée pour être déformée dans la seconde direction et la seconde plaque (750) est configurée pour être déformée dans la première direction en réponse à une contraction de l'élément piézoélectrique (710) afin d'augmenter une distance entre la première plaque (740) et la seconde plaque (750) ;
le diaphragme (720) est configuré pour être déformé dans la première direction en réponse à une dilatation de l'élément piézoélectrique (710) ; et
le diaphragme (720) est configuré pour être déformé dans la seconde direction en réponse à une contraction de l'élément piézoélectrique (710).

12. Dispositif gonflable piloté par un fluide et implantable (100 ; 100A ; 100B) selon la revendication 1, dans lequel l'élément piézoélectrique (810) inclut :
une pile de couches piézoélectriques qui inclut une pluralité de couches piézoélectriques agencées séquentiellement suivant une surface de montage du diaphragme (820), depuis une première partie d'extrémité jusqu'à une seconde partie d'extrémité de la pile de couches piézoélectriques ;
une première console de support (840) qui couple une première couche piézoélectrique, au niveau de la première partie d'extrémité de la pile de couches piézoélectriques, à une surface de montage du diaphragme (820) ; et
une seconde console de support (850) qui couple une seconde couche piézoélectrique, au niveau de la seconde partie d'extrémité de la pile de couches piézoélectriques, à la surface de montage du diaphragme (820).

13. Dispositif gonflable piloté par un fluide et implantable (100 ; 100A ; 100B) selon la revendication 12, dans lequel :
la pile de couches piézoélectriques est configurée pour être dilatée en réponse à une première tension qui lui est appliquée ;
la première partie d'extrémité de la pile de couches piézoélectriques est configurée pour pivoter dans une première direction de pivotement au niveau de la première console de support (840) en réponse à une dilatation de la pile de couches piézoélectriques ; et
la seconde partie d'extrémité de la pile de couches piézoélectriques est configurée pour pivoter dans une seconde direction de pivotement au niveau de la seconde console de support (850) en réponse à une dilatation de la pile de couches piézoélectriques.

14. Dispositif gonflable piloté par un fluide et implantable (100) selon la revendication 12 ou la revendication 13, dans lequel :
la pile de couches piézoélectriques est configurée pour être contractée en réponse à une seconde tension qui lui est appliquée ;
la première partie d'extrémité de la pile de couches piézoélectriques est configurée pour pivoter dans la seconde direction de pivotement au niveau de la première console de support (840) en réponse à une contraction de la pile de couches piézoélectriques ; et
la seconde partie d'extrémité de la pile de couches piézoélectriques est configurée pour pivoter dans la première direction de pivotement au niveau de la seconde console de support (850) en réponse à une contraction de la pile de couches piézoélectriques.

15. Dispositif gonflable piloté par un fluide et implantable (100 ; 100A ; 100B) selon l'une quelconque des revendications 12 à 14, dans lequel :
le diaphragme (820) est configuré pour être déformé dans une première direction en réponse à une dilatation de la pile de couches piézoélectriques ; et
le diaphragme (820) est configuré pour être déformé dans une seconde direction en réponse à une contraction de la pile de couches piézoélectriques, et dans lequel un espace (815) est formé entre une partie de fond de l'élément piézoélectrique (810) et la surface de montage du diaphragme (820) et dans lequel une distance entre la partie de fond de l'élément piézoélectrique (810) et la surface de montage du diaphragme (820) augmente en réponse à une dilatation de la pile de couches piézoélectriques et diminue en réponse à une contraction de la pile de couches piézoélectriques.
